# FASCICULE DE BREVET EUROPEEN

(11) **EP 1 185 662 B1**
(45) Date de publication et mention de la délivrance du brevet: **02.03.2011**
(21) Numéro de dépôt: 00940474.0
(22) Date de dépôt: 08.06.2000
(51) Int. Cl.: A61K 39/175, C12N 15/38, C12N 15/27, A61K 48/00

(54) **VACCINS ADN POUR LES CHIENS**
DNS-IMPFSTOFFE FÜR HUNDE
DNA VACCINES FOR DOGS

(30) Priorité: 10.06.1999 FR 9907604; 19.07.1999 US 144490 P
(43) Date de publication de la demande: 13.03.2002
(73) Titulaire: MERIAL, 69007 Lyon (FR)
(72) Inventeur: FISCHER, Laurent, Jean-Charles, F-69110 Sainte-Foy Les Lyon (FR); BARZU-LE ROUX, Simona, F-69210 Lentilly (FR); AUDONNET, Jean-Christophe, Francis, F-69006 Lyon (FR)
(74) Mandataire: Nargolwalla, Cyra
(86) Numéro de dépôt international: PCT/FR2000/001592
(87) Numéro de publication internationale: WO 2000/077043

(56) Documents cités:
- WO-A-00/24428
- WO-A-96/34109
- WO-A-97/41236
- WO-A-98/03198
- WO-A-98/03199
- WO-A-98/03660
- WO-A-98/40499
- NASH ET AL: "MOLECULAR CLONING AND IN VIVO EVALUATION OF CANINE GRANULOCYE-MACROPHAGE COLONY-STIMULATING FACTOR" PUBMED, NATIONAL LIBRARY OF MEDICINE, [Online] XP002133949 Retrieved from the Internet: <URL:http://www.ncbi.nlm.nih.gov:80/entrez /query.fcgi?cmd=Retrieve&db=PubMed&list_ui ds=1868252&dopt=Abstract> [retrieved on 2000-03-23] & BLOOD, vol. 78, 1991, pages 930-937,
- DATABASE BIOSIS [Online] BIOSCIENCES INFORMATION SERVICE, PHILADELPHIA, PA, US; 1997 NAIK SANGEETA ET AL: "Characterization of membrane-bound and membrane anchor-less forms of hemagglutinin glycoprotein of rinderpest virus expressed by baculovirus recombinants." Database accession no. PREV199799657927 XP002133952 & VIRUS GENES 1997, vol. 14, no. 2, 1997, pages 95-104, ISSN: 0920-8569
- GAO YI ET AL: "Truncated bovine herpesvirus-1 glycoprotein I (gpI) initiates a protective local immune response in its natural host." VACCINE 1994, vol. 12, no. 2, 1994, pages 145-152, XP002133950 ISSN: 0264-410X
- DATABASE BIOSIS [Online] BIOSCIENCES INFORMATION SERVICE, PHILADELPHIA, PA, US; 1997 HADDAD DIANA ET AL: "Comparative study of DNA-based immunization vectors: Effect of secretion signals on the antibody responses in mice." Database accession no. PREV199799707402 XP002133953 & FEMS IMMUNOLOGY AND MEDICAL MICROBIOLOGY 1997, vol. 18, no. 3, 1997, pages 193-202, ISSN: 0928-8244
- LI XIAOMAO ET AL: "Protection against respiratory syncytial virus infection by DNA immunization." JOURNAL OF EXPERIMENTAL MEDICINE AUG. 17, 1998, vol. 188, no. 4, 17 August 1998 (1998-08-17), pages 681-688, XP002133951 ISSN: 0022-1007
- VACCINE, vol. 21, 2003, pages 4593-4596,
- VETERINARY IMMUNOLOGY AND IMMUNOPATHOLOGY, vol. 111, 2006, pages 47-57,
- J. IMMUNOLOGY, vol. 159, 1997, pages 3638-3647,
- J. IMMUNOLOGY, vol. 162, 1 January 1999 (1999-01-01), pages 254-262,
- J. BIOL. CHEM., vol. 269, 1994, pages 2550-2561,

## Description

La présente invention a trait à des vaccins ADN améliorés pour les chiens.

L'utilisation de molécules d'acide désoxyribonucléique (ADN) pour la vaccination est connue depuis le début des années 90 (Wolf et al. Science 1990. 247. 1465-1468). Cette technique de vaccination induit une immunité cellulaire et humorale après la transfection *in vivo* de cellules du sujet à vacciner par des molécules d'ADN ou d'ARN codant et exprimant des protéines immunologiquement actives.

Un vaccin ADN se compose d'au moins un plasmide pouvant être exprimé par la machinerie cellulaire du sujet à vacciner et d'un véhicule ou d'un excipient pharmaceutiquement acceptable. La séquence nucléotidique de ce plasmide code et exprime, entre autres, un ou plusieurs immunogènes, tels que des protéines ou glycoprotéines capables d'induire, chez le sujet à vacciner, une réponse immunitaire cellulaire (mobilisation des lymphocytes T) et humorale (stimulation de la production d'anticorps spécifiquement dirigés contre l'immunogène) (Davis H. L. Current Opinion Biotech. 1997.8. 635-640).

Tous les immunogènes provenant d'un pathogène ne sont pas des antigènes assez efficaces naturellement pour induire une réponse immunitaire protectrice optimale chez l'animal à vacciner. II est donc nécessaire d'améliorer la réponse immunitaire.

Chaque voie d'administration comporte ses propres contraintes et difficultés ; ainsi un vaccin ADN efficace par une voie d'administration peut être inefficace par une autre.

Le choix de la voie d'administration doit tenir compte des besoins des praticiens et des éleveurs, des difficultés liées à la contention des animaux ou à la nature du produit.

Bien que la voie intramusculaire puisse être utilisée, la voie sous-cutanée présente un grand intérêt pour la vaccination des animaux de compagnie, surtout pour les animaux de petite taille et de manipulation difficile.

Les vaccins ADN doivent donc être améliorés pour permettre leur administration efficace par des voies différentes.

Des vaccins ADN ont déjà été utilisés expérimentalement, notamment un vaccin ADN codant pour l'hémagglutinine (HA) du virus de la rougeole (Etchart et al. J. Gen. Virol. 1997. 78. 1577-1580) dont l'administration intranasale chez la souris s'est révélée être plus efficace qu'une administration orale. Autre exemple, un vaccin ADN codant pour la protéine d'enveloppe (Env) du virus de l'immunodéficience humaine (HIV) dont l'administration sous-cutanée n'a pas été efficace en comparaison de l'administration par la voie intramusculaire (Ishii et al. AIDS Res. Hum. Retro. 1997. 13. 1421-1428).

Les vaccins ADN ont également été utilisés expérimentalement contre des virus d'animaux, notamment contre les virus de la maladie de Carré (CDV). Certains immunogènes de CDV sont connus, notamment la protéine de nucléocapside (N), la protéine de matrice (M), la protéine de fusion (F) et l'hemagglutinine (HA) (WO-A-9741236). Cependant, l'administration sous-cutanée d'un vaccin ADN codant pour l'hémagglutinine et la protéine de fusion de CDV n'a pas permis de détecter la production d'anticorps chez la .souris, et uniquement une faible production d'anticorps après l'administration intramusculaire de ce vaccin ADN (Sixt et al. J. Virol. 1998. 72. 8472-8476).

L'induction d'une réponse immunitaire et les relations entre les différents éléments du système immunitaire intervenant lors de cette réponse peuvent être différentes d'une espèce animale à une autre. Les nombreux enseignements tirés des expériences menées sur le modèle souris ont permis de mieux comprendre le fonctionnement du système immunitaire chez la souris, mais ces enseignements ne sont pas directement transposables aux autres espèce, notamment car il est plus facile d'induire une réponse immunitaire chez la souris que chez les autres espèces (van Drunen Little-van den Hurk et al. J. Gen. Virol. 1998. 79. 831-839 ; Bôhm et al. Vaccine 1998. 16. 949-954).

Différentes voies d'administration du vaccin ADN ont été proposées (intrapéritonéale, intraveineuse, intramusculaire, sous-cutanée, intradermique, muqueuse, etc.). Différents moyens d'administration ont également été proposés, notamment des particules d'or enrobées d'ADN et projetées de façon à pénétrer dans les cellules de la peau du sujet à vacciner (Tang et al. Nature 1992. 356. 152-154) et les injecteurs par jet liquide permettant de transfecter à la fois des cellules de la peau et des cellules des tissus sous-jacents (Furth et al. Analytical Bioch. 1992. 205. 365-368).

Des composés chimiques ont été utilisés pour la transfection *in vitro* d'ADN :
A/ - les lipides cationiques.
   Les lipides cationiques sont eux-mêmes divisés en quatre sous-groupes.
   1) les lipides cationiques contenant des sels d'ammonium quaternaire, comme par exemple le DOTMA (dioléoytoxypropyl-triméthylammonium, produit par Gibco sous le nom de Lipofectine), le DOTAP (triméthyl-2,3-(octadéc-9-ène-oyloxy)-1-propanammonium; Gregoriadis et al. FEBS Letters 1997. 402. 107-110), le DMRIE (N-(2-hydroxyéthyl)-N,N-diméthyl-2,3-bis(tetradécyloxy)-1-propanammonium ; WO-A-9634109), le DLRIE (N-(2-hydroxyéthyl)-N,N-diméthyl-2,3-bis(dodécyloxy)-l-propanammonium ; Felgner et al. Ann. N Y Acad. Sci. 1995. 772. 126-139).
      Ces lipides cationiques contenant des sels d'ammonium quaternaire peuvent être associés ou non avec un lipide neutre supplémentaire, tel que le DOPC (dioléoyl-phosphatidyl-choline) ou le DOPE (dioléoyl-phosphatidyl-éthanolamine) (J.P. Behr, Bioconjugate Chemistry 1994. 5. 382-389).
   2) les lipoamines, comme par exemple le DOGS (dioctadécylamidoglycylspermine, produit par Promega sous le nom de Transfectam; Abdallah et al. Biol. Cell. 1995. 85. 1-7), le DC-Chol (diméthylaminoéthane-carbamoyl-cholestérol ; Gao et Huang, Biochem. Biophys. Res. Commun. 1991. 179. 280-285), le BGSC (bis-guanidine-spermidine-cholestérol), le BGTC (bis-guanidine-tren-cholestérol) (Vigneron et al. Proc. Natl. Acad. Sci. USA 1996. 93. 9682-9686).
   3) les lipides cationiques contenant des sels d'ammonium quaternaire et des lipoamines, comme par exemple le DOSPA (N,N-diméthyl-N-(2-(sperminecarboxamido)éthyl)-2,3-bis(dioléoyloxy)-1-propanimidium pentahydrochloride, commercialisé par Gibco sous le nom de LipofectAmine® ; Hawley-Nelson et al. Focus 1993. 15. 73-79), le GAP-DLRIE (N-(3-aminopropyl)-N,N-diméthyl-2,3-bis(dodécyloxy)-i-propanaminium ; Wheeler et al. Proc. Natl. Acad. Sci. USA 1996. 93. 11454-11459 ; Norman et al. Vacine 1997. 15. 801-803).
   4) les lipides contenant des sels d'amidine, comme par exemple l'ADPDE, l'ADODE (Ruysschaert et al. Biochem. Biophys. Res. Commun. 1994. 203. 1622-1628).
B/ - les polymères, comme par exemple le SuperFect™ (molécules de dendrimères activés, produits par Qiagen ; Xu et al. Mol. Genet. Metab. 1998. 64. 193-197), et
C/ - les agents biochimiques, comme par exemple les toxines, notamment les toxines cholériques.

Certains de ces composés ont aussi été utilisés dans la formulation de vaccins ADN avec des résultats plus que mitigés. Les connaissances en matière de transfection *in vitro* ne sont pas transposables à la vaccination ADN où l'objectif final est d'assurer une réaction immunitaire protectrice. Des effets négatifs sur l'induction d'une protection immunitaire efficace ont même été constatés avec des composés connus pour favoriser la transfection *in vitro.* Certains composés chimiques de formulation sont toxiques à hautes doses pour les cellules transfectées.

Dans les travaux de Etchart déjà cités (Etchart et al. J. Gen. Virol. 1997. 78. 1577-1580), l'utilisation du DOTAP n'a pas eu d'effet adjuvant lors de l'administration du vaccin ADN par la voie intranasale, alors qu'il en a eu par la voie orale. Le DOTAP a également été utilisé dans des vaccins ADN codant pour l'hémagglutinine (HA) du virus de la grippe sur le modèle souris administrés par la voie intranasale (Ban et al. Vaccine 1997. 15. 811-813), mais l'addition de DOTAP a inhibé la réponse immunitaire. L'utilisation de DC-Chol ou de DOTAP/DOPE dans des vaccins ADN codant pour la protéine de surface (S) du virus de l'hépatite B sur le modèle souris administrés par la voie intramusculaire a permis d'augmenter la réponse en anticorps, alors que l'utilisation de la Lipofectine (ou DOTMA) n'a pas augmenté cette réponse (Gregoriadis et al. FEBS Letters 1997. 402. 107-110). Le DC-Chol/DOPE a également été utilisé dans des vaccins ADN contre le virus de l'immunodéficience humaine (HIV, protéine Env) sur le modèle souris, dont l'administration par la voie intramusculaire a induit une réponse immunitaire plus efficace, alors que l'administration par la voie sous-cutanée ou intradermique ne l'a pas augmentée (Ishii et al. AIDS Res. Hum. Retro. 1997. 13. 1421-1428).

De même, WO-A-98 40499 propose de réaliser des complexes acide nucléique + lipides cationiques pour transfecter l'épithélium mucosal des mammifères, pour de la thérapie génique ou l'expression d'antigène destiné à induire une réponse immunitaire. Ce document vise la voie mucosale, par inhalation, par exemple l'épithélium pulmonaire. Il précise que ses résultats divergent de travaux antérieurs. Et il ajoute que pour la voie intramusculaire (parentérale), l'ADN nu est plus efficace qu'un mélange ADN + lipide.

L'addition de certaines cytokines, notamment d'interleukines ou d'interférons, peut permettre d'améliorer la réponse immunitaire induite en particulier par les vaccins ADN. Chaque cytokine déclenche une réaction qui lui est propre et oriente plus ou moins la réponse immunitaire vers une réponse cellulaire ou vers une réponse humorale (Pasquini et al. Immunol. Cell. Biol. 1997. 75. 397-401 ; Kim et al. J. Interferon Cytokine Res. 1999. 19. 77-84). Les effets adjuvants d'une cytokine provenant d'une espèce donnée ne sont pas nécessairement les mêmes si le contexte immunitaire varie, notamment si cette cytokine est administrée à une autre espèce, donc dans un système immunitaire hétérologue. L'addition de cytokine peut également n'avoir aucun effet adjuvant, voire aboutir à une inversion de l'effet recherché, c'est-à-dire une diminution ou une inhibition de la réponse immunitaire. Ainsi, un vaccin ADN codant pour une chaîne simple d'une immunoglobuline fusionnée avec le GM-CSF n'augmente pas la réponse immunitaire, alors que l'administration directe chez la souris de cette protéine de fusion est efficace, tout comme l'est l'administration d'une protéine de fusion formée de Fv et de la cytokine IL-1bêta ou l'administration d'un vaccin ADN codant pour cette dernière protéine de fusion (Hakim et al. J. Immunol 1996. 157. 5503-5511). L'utilisation de plasmides co-exprimant la cytokine IL-2 et la protéine d'enveloppe du virus de l'hépatite B dans une conformation fusionnée ou non fusionnée résulte dans l'augmentation des réponses immunitaires humorales et cellulaires (Chow et al. J. Virol. 1997. 71. 169-78). Mais l'utilisation d'un plasmide bicistronique codant pour la glycoprotéine gp120 du virus de l'immunodéficience acquise humaine (HIV-1) et la cytokine IL-2 a induit une réponse immunitaire spécifique anti-gp120 plus faible que celle obtenue par l'utilisation d'un plasmide monocistronique codant uniquement pour gp120 (Barouch et al. J. Immunol 1998. 161. 1875-1882). La co-injection chez la souris de deux vecteurs d'expression, l'un codant pour la glycoprotéine G du virus de la rage, l'autre pour le GM-CSF murin stimule l'activité des lymphocytes B et T, alors que la co-injection avec un plasmide codant pour l'interféron gamma (à la place du GM-CSF murin) a pour résultat une diminution de la réponse immunitaire (Xiang et al. Immunity 1995. 2. 129-135).

Certaines modifications au niveau des antigènes, telles que les délétions d'une partie de la séquence nucléotidique codant pour l'antigène, les insertions d'un fragment d'ADN dans la séquence nucléotidique codant pour l'antigène ou dans les régions non traduites en amont ou en aval, peuvent également améliorer l'efficacité des vaccins ADN, notamment en améliorant le niveau d'expression de l'antigène ou sa présentation.

Mais en pratique, les manipulations au niveau de la séquence nucléotidique codant pour l'antigène peuvent entraîner une diminution ou la perte de l'activité immunologique initiale. Ainsi, la délétion du domaine transmembranaire dans le gène codant pour l'antigène G du virus de la rage a diminué le niveau de protection induit chez le modèle souris après administration par la voie intramusculaire d'un vaccin ADN codant pour cet antigène modifié (Xiang *et al.* Virol. 1995. **209**. 569). La délétion du domaine transmembranaire dans le gène codant pour la glycoprotéine gD du virus herpès bovin (BHV) n'a pas permis d'augmenter la réponse en anticorps et n'a induit qu'une protection partielle chez les bovins vaccinés par la voie intramusculaire (van Drunen Little-van den Hurk et al. J. Gen. Virol. 1998. 79. 831-839). Les réponses immunitaires humorales et cellulaires et la protection conférée sont identiques chez des cobayes éprouvés après avoir été immunisés à l'aide soit d'un vaccin ADN codant pour la glycoprotéine GP du virus Ebola, soit d'un vaccin ADN codant pour cette glycoprotéine GP mais sous une forme sécrétée (Xu et al. Nature Medicine 1998. 4. 37-42).

L'insertion de la séquence signal de l'activateur plasminogène tissulaire humain (en anglais human tissue Plasminogen Activator ou human tPA) dans le gène codant pour l'antigène Pf332 de la malaria n'a pas permis d'augmenter la réponse en anticorps chez la souris vaccinée par voie intramusculaire (Haddad et al. FEMS 1997. 18. 193-202). L'addition en phase d'une séquence tPA au gène codant pour l'antigène VP7 du rotavirus murin n'a pas également permis d'augmenter la réponse en anticorps chez la souris vaccinée par voie intradermique, alors que la protéine de fusion formée de l'antigène VP4 et du tPA a permis cette augmentation, mais sans induire une protection efficace (Choi et al. Virology 1998. 250. 230-240).

Les modifications effectuées sur la séquence nucléotidique d'un antigène ne peuvent pas en général être directement transposées à un autre antigène, car les antigènes n'ont pas toujours les mêmes agencements structuraux.

La déposante a pour objectif l'amélioration de l'efficacité de la vaccination ADN. Elle a en particulier pour objectif d'obtenir une meilleure réponse immunitaire et notamment une protection efficace chez le chien, par la vaccination ADN, pour différentes voies d'administration, et en particulier pour la voie sous-cutanée.

La déposante a pour objectif l'élaboration de vaccins ADN améliorés induisant une réponse immunitaire efficace et protectrice contre le virus de la maladie de Carré (CDV) chez le chien.

La déposante a également pour objectif l'élaboration de vaccins ADN améliorés permettant d'obtenir une protection efficace chez le chien, comprenant une valence formé du virus CDV.

L'invention a pour objet des vaccins ADN améliorés permettant d'obtenir une protection efficace contre au moins un pathogène infectant les chiens.

Le vaccin ADN est amélioré par sa formulation, et facultativement soit par l'addition de GM-CSF, soit par l'optimisation du ou des antigènes, enfin soit par l'addition de GM-CSF et par l'optimisation du ou des antigènes.

Par définition, le vaccin ADN comprend comme principe actif un plasmide codant pour et exprimant un gène ou fragment de gène. Le terme plasmide recouvre une unité de transcription ADN comprenant une séquence polynucléotidique comprenant la séquence du gène à exprimer et les éléments nécessaires à son expression *in vivo*. On préfére la forme plasmide circulaire, superenroulée ou non. La forme linéaire entre également dans le cadre de cette invention.

Chaque plasmide comprend un promoteur apte à assurer, dans les cellules hôtes, l'expression du gène inséré sous sa dépendance. Il s'agit en général d'un promoteur eucaryote fort et en particulier d'un promoteur précoce du cytomégalovirus CMV-IE, d'origine humaine ou murine, ou encore éventuellement d'une autre origine telle que rat, cobaye. De manière plus générale, le promoteur est soit d'origine virale, soit d'origine cellulaire. Comme promoteur viral autre que CMV-IE, on peut citer le promoteur précoce ou tardif du virus SV40 ou le promoteur LTR du virus du Sarcome de Rous. Il peut aussi s'agir d'un promoteur de virus dont provient le gène, par exemple le promoteur propre du gène. Comme promoteur cellulaire, on peut citer le promoteur d'un gène du cytosquelette, tel que par exemple le promoteur de la desmine, ou encore le promoteur de l'actine. Lorsque plusieurs gènes sont présents dans le même plasmide, ceux-ci peuvent être présentés dans la même unité de transcription ou dans deux unités différentes.

Suivant une première modalité, les vaccins ADN selon l'invention sont formulés par addition à titre d'adjuvant, le DMRIE, associé avec le DOPE.

La présente invention a donc pour objet un vaccin ADN contre au moins un pathogène touchant les chiens, comprenant au moins un plasmide contenant au moins une séquence nucléotidique codant pour un immunogène d'un pathogène de l'espèce animale considérée, dans des conditions permettant l'expression *in vivo* de cette séquence, et le DMRIE (N-(2-hydroxyéthyl)-N,N-diméthyl-2,3-bis(tetradécyloxy)-1-propanammonium ; WO-A-9634109) associé avec le DOPE (dioléoyl-phosphatidyl-éthanolamine).

De préférence, le mélange vecteur recombinant avec cet adjuvant se fait de manière extemporanée et l'on préfère, avant son administration à l'animal, laisser le temps au mélange ainsi constitué de se complexer, par exemple pendant une durée allant de 10 à 60 minutes, notamment de l'ordre de 30 minutes.

Le ratio molaire DMRIE : DOPE va de préférence de 95 : 5 à 5 : 95, plus particulièrement de 1 : 1.

Le ratio pondéral plasmide : adjuvant DMRIE-DOPE peut aller notamment de 50 : 1 à 1 : 10, notamment de 10 : 1 à 1 : 5, de préférence de 1 : 1 à 1 : 2.

Suivant une deuxième modalité, on ajoute aux vaccins selon l'invention le GM-CSF (en anglais Granulocyte macrophage - colony stimulating factor ; Clark S. C. et al. Science 1987. 230. 1229 ; Grant S. M. et al. Drugs 1992. 53. 516), ce qui peut se faire par l'incorporation de protéine GM-CSF directement dans la composition vaccinale ou de préférence par l'insertion de la séquence codant pour le GM-CSF dans un vecteur d'expression dans des conditions permettant son expression *in vivo.* Comme vecteur d'expression, on préfère utiliser un plasmide, e.g. le plasmide contenant la séquence nucléotidique codant pour le (ou les) antigène(s) d'intérêt ou un autre plasmide. Le choix du GM-CSF se fait en fonction de l'espèce animale à vacciner, ainsi pour les chiens le GM-CSF canin est utilisé.

Suivant une troisième modalité, la (ou les) séquence nucléotidique codant pour l'immunogène sont sous une forme optimisée. Par optimisation, on entend toute modification de la séquence nucléotidique, notamment se manifestant au moins par un niveau d'expression plus élevé de cette séquence nucléotidique, par une augmentation de la stabilité de l'ARN messager codant pour cet antigène, par la sécrétion provoquée de cet antigène dans le milieu extracellulaire, et ayant pour conséquence directe ou indirecte une augmentation de la réponse immunitaire induite.

Dans la présente invention, l'optimisation de l'antigène d'intérêt consiste de préférence en la délétion du fragment de la séquence nucléotidique codant pour le domaine transmembranaire de l'antigène d'intérêt (par délétion, on entend la délétion totale ou une délétion partielle suffisante pour que le domaine transmembranaire ne soit plus, ou substantiellement plus, fonctionnel), et/ou en l'addition en phase d'une séquence nucléotidique codant pour le signal tPA (en anglais tissue plasminogen activator ; Montgomery et al. Cell. Mol. Biol. 1997. 43. 285-292 ; Harris et a/. Mol. Biol. Med 1986. 3. 279-292), et/ou en l'insertion d'intron stabilisateur en amont du gène à exprimer. La délétion du fragment d'ADN codant pour le domaine transmembranaire de l'antigène d'intérêt favorise la sécrétion vers le milieu extracellulaire des antigènes ainsi tronqués et ainsi augmente leurs possibilités de contact avec les cellules du système immunitaire. L'insertion de la séquence nucléotidique codant pour le signal tPA facilite la traductibilité de l'ARN messager auquel le signal tPA est joint, et augmente ainsi le niveau d'expression de cet ARN messager et donc la production d'antigènes. Le signal tPA joue aussi un rôle dans la sécrétion de l'antigène synthétisé. L'insertion d'un intron stabilisateur dans le gène codant pour l'antigène d'intérêt évite les épissages aberrants de son ARN messager et maintient l'intégrité physique de ce dernier.

De préférence le signal tPA est d'origine humaine. La séquence nucléotidique du signal tPA humain est accessible auprès de la base de données Genbank sous le numéro d'accès NM_000930. De préférence, l'intron est l'intron Il du gène de la bêta-globine du lapin (van Ooyen et al. Science 1979. 206. 337-344), dont la séquence nucléotidique est accessible auprès de la base de données GenBank sous le numéro d'accès V00882 et référencée sous intron n°2.

La présente invention a pour objet un vaccin ADN amélioré capable d'induire une réponse immunitaire efficace et protectrice chez le chien contre la maladie de Carré (en anglais Canine Distemper Virus ou CDV).

Le virus de la maladie de Carré est un *Morbillivirus,* membre de la famille des *Paramyxoviridae.* Ce virus infecte l'espèce canine, mais aussi les félins sauvages (Harder et al. J. Gen. Virol. 1996. 77. 397-405).

La présente invention permet d'obtenir un vaccin ADN efficace et protecteur contre la maladie de Carré chez le chien, en particulier par la voie sous-cutanée qui demeurait jusqu'à ce jour une voie induisant un niveau de protection marginal (Sixt et al. J. Virol. 1998. 72. 8472-8476).

Selon l'invention, le vaccin ADN contre CDV est amélioré par sa formulation avec un adjuvant selon l'invention, le DMRIE-DOPE. Facultativement, cela peut être combiné avec soit l'addition de GM-CSF canin (Nash et al. Blood 1991. 78. 50-56), soit l'optimisation d'au moins un antigène de CDV, enfin soit l'addition de GM-CSF canin et l'optimisation d'au moins un antigène de CDV.

Une séquence nucléotidique codant pour le GM-CSF canin est accessible auprès de la base de données GenBank sous le numéro d'accès S49738.

L'addition de GM-CSF canin peut se faire par l'incorporation du polypeptide GM-CSF canin dans la composition vaccinale ou de préférence par l'insertion de la séquence nucléotidique codant pour le GM-CSF canin dans un vecteur d'expression *in vivo,* de préférence un plasmide. De préférence, la séquence nucléotidique codant pour le GM-CSF canin est insérée dans un second plasmide d'expression, différent de celui (ou de ceux) dans lequel est inséré le ou les gènes codant pour le ou les antigènes de CDV.

L'optimisation des antigènes issus de CDV se fait par substitution, par une séquence " signal ", notamment celle du signal tPA d'origine humaine (GenBank numéro d'accès NM_000930), de la séquence du peptide signal de l'hémagglutinine (HA) et/ou de la protéine de fusion (F), et/ou par la délétion du fragment d'ADN codant pour le domaine transmembranaire de HA et/ou de F, et/ou par l'insertion d'un intron, notamment de l'intron II du gène de la bêta-globine du lapin (dont la séquence nucléotidique, notée intron n°2, est accessible auprès de la base de données GenBank sous le numéro d'accès V00882) en amont de la séquence nucléotidique codant pour HA et/ou F. Le vaccin ADN contre CDV selon l'invention peut donc coder et exprimer un seul antigène de CDV optimisé (HA ou F) ou les deux, c'est-à-dire HA optimisé et F optimisé.

Optionnellement, la séquence codant pour la protéine de matrice (M) de CDV sous sa forme native (sans modification) et/ou la séquence nucléotidique codant pour la nucléoprotéine (N) de CDV sous sa forme native (sans modification) peuvent être également insérées et exprimées dans un plasmide et associées aux plasmides contenant HA optimisé et/ou F optimisé.

Des séquences nucléotidiques codant pour les antigènes de CDV utilisable dans la présente invention et différentes constructions de vecteurs d'expression sont illustrées dans les exemples annexés et dans WO-A-9803199, en particulier dans ses exemples 8 et 9 et ses figures 2 et 3.

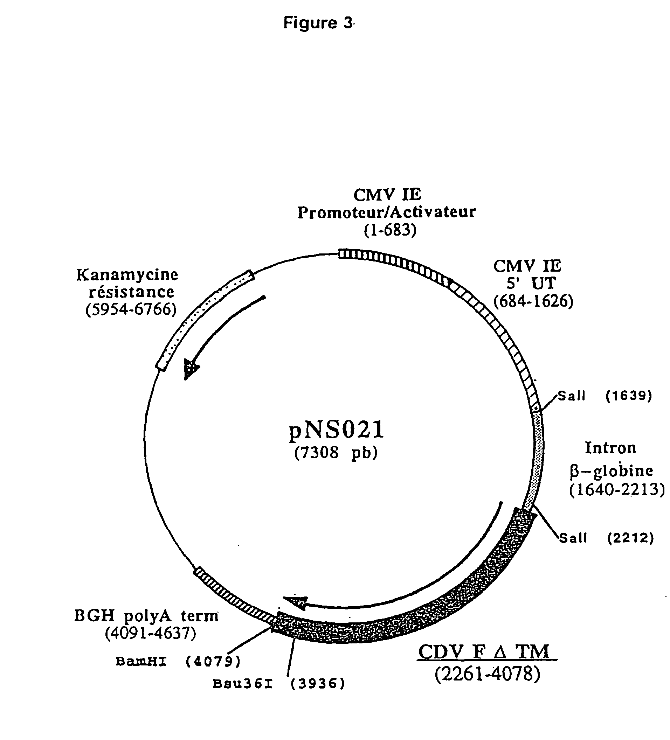

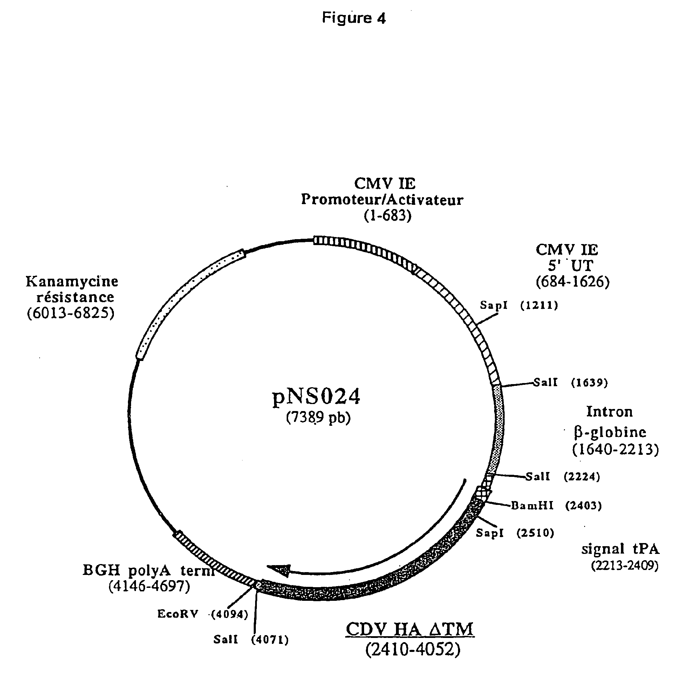

De manière préférentielle selon l'invention, le vaccin ADN contre CDV en vue d'une administration par la voie intramusculaire est formulé avec du DMRIE-DOPE, et se compose d'un plasmide d'expression (e.g. pNS024, Figure 4) codant pour l'antigène HA de CDV optimisé par substitution de la séquence signal de HA par la séquence peptide signal tPA humain, par la délétion du fragment de la séquence nucléotidique codant pour le domaine transmembranaire de HA et par l'insertion de l'intron 11 du gène de la bêta-globine du lapin en amont du gène HA, et d'un second plasmide d'expression (e.g. pNS021, Figure 3) codant pour l'antigène F de CDV optimisé par la délétion du fragment de la séquence nucléotidique codant pour le domaine transmembranaire et par l'insertion de l'intron II du gène de la bêta-globine du lapin en amont du gène F.

De manière préférentielle selon l'invention, le vaccin ADN contre CDV en vue d'une administration par la voie sous-cutanée est formulé avec du DMRIE-DOPE, et se compose d'un plasmide d'expression codant pour le GM-CSF canin et des deux plasmides précédemment définis (e.g. pNS024 et pNS021).

Les vaccins selon l'invention peuvent être, pour une espèce animale, combinés avec des vaccins ADN dirigés contre d'autres pathogènes de la même espèce.

Ainsi, la présente invention a également pour objet des vaccins ADN multivalents améliorés permettant d'obtenir une protection efficace chez le chien contre au moins CDV et un pathogène canin choisis parmi le groupe formé de : CPI-2, CHV-1, virus de la rage (*rhabdovirus*)*,* parvovirus canin (CPV), coronavirus canin (CCV), *Borrelia burgdorferi.*

Les vaccins ADN multivalents sont améliorés par leur formulation avec un adjuvant selon l'invention, le DMRIE-DOPE. Ceci peut être facultativement combiné soit avec l'addition de GM-CSF comme cela est précédemment décrit, soit avec l'optimisation d'au moins un antigène d'intérêt comme cela est précédemment décrit, enfin soit par l'addition de GM-CSF et l'optimisation d'au moins un antigène d'intérêt.

Les vaccins ADN multivalents améliorés selon l'invention se composent d'un ou plusieurs plasmides d'expression, de telle façon que ces vaccins conduisent à l'expression *in vivo* d'au moins un immunogène d'un premier pathogène et d'au moins un immunogène d'au moins un autre pathogène, infectant la même espèce animale. L'un au moins de ces immunogènes est de préférence choisi parmi les membres du groupe suivant :
- F de CDV et HA de CDV, pour les chiens.

Les vaccins ADN monovalents ou multivalents améliorés selon l'invention peuvent être également associés avec au moins un vaccin classique (inactivé, vivant atténué, sous-unités) ou vaccin recombiné utilisant un vecteur d'expression *in vivo* (e.g. poxvirus, adénovirus, herpèsvirus) dirigé contre au moins un pathogène notamment différent infectant la même espèce.

L'homme de l'art peut se reporter à WO-A-9803198 pour les méthodes pour construire les plasmides contenant ces valences équines, à WO-A-9803660 pour les valences félines et à WO-A-9803199 pour les valences canines.

La quantité d'ADN utilisée dans les vaccins selon la présente invention est comprise entre environ 10 µg et environ 1000 µg, et préférentiellement entre environ 50 µg et environ 500 µg, pour un plasmide donné. L'homme de l'art possède les compétences nécessaires pour définir précisément la dose efficace d'ADN à utiliser pour chaque protocole de vaccination.

Les volumes de dose peuvent être de préférence compris entre 0,5 et 5 ml, de préférence entre 1 et 3 ml.

Les vaccins ADN améliorés selon l'invention peuvent être administrés, dans le cadre de cette méthode de vaccination, par les différentes voies d'administration proposées dans l'art antérieur pour la vaccination polynucléotidique et au moyen des techniques d'administration connues.

Suivant les deux modalités préférées de l'invention, les méthodes de vaccination comprennent l'administration par la voie intramusculaire ou par la voie sous-cutanée des vaccins ADN améliorés selon l'invention.

L'invention va être maintenant décrite plus en détail à l'aide de modes de réalisation pris à titre d'exemples non limitatifs et se référant au dessin dans lequel :
Figure N° 1 : Plasmide pAB110
Figure N° 2 : Plasmide pVR1012
Figure N° 3 : Plasmide pNS021
Figure N° 4 : Plasmide pNS024
Figure N° 5 : Plasmide pSB032
Figure N° 6 : Plasmide pSB034
Figure N° 7 : Plasmide pSB016
Figure N° 8 : Plasmide pSB019
Figure N° 9 : Plasmide pSB017
Figure N° 10 : Plasmide pSB021
Figure N° 11 : Plasmide pSB023
Figure N° 12 : Plasmide pSB024

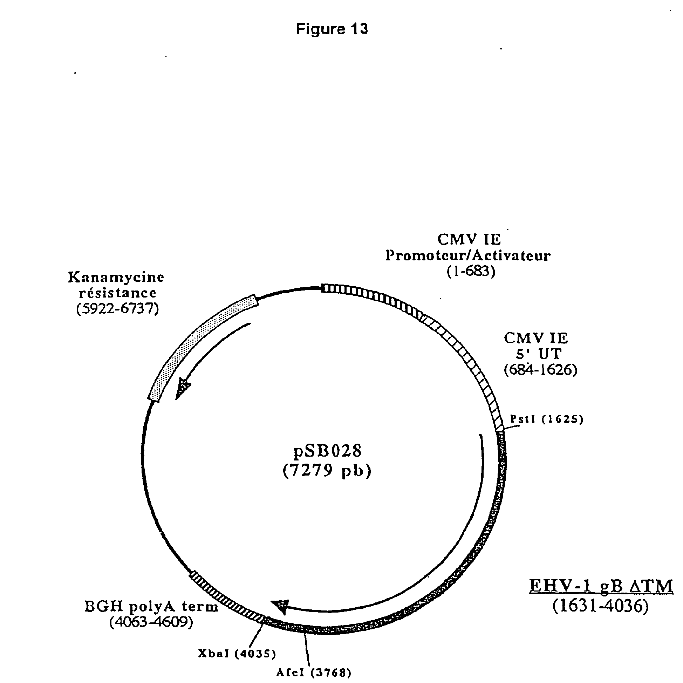

Figure N° 13 : Plasmide pSB028

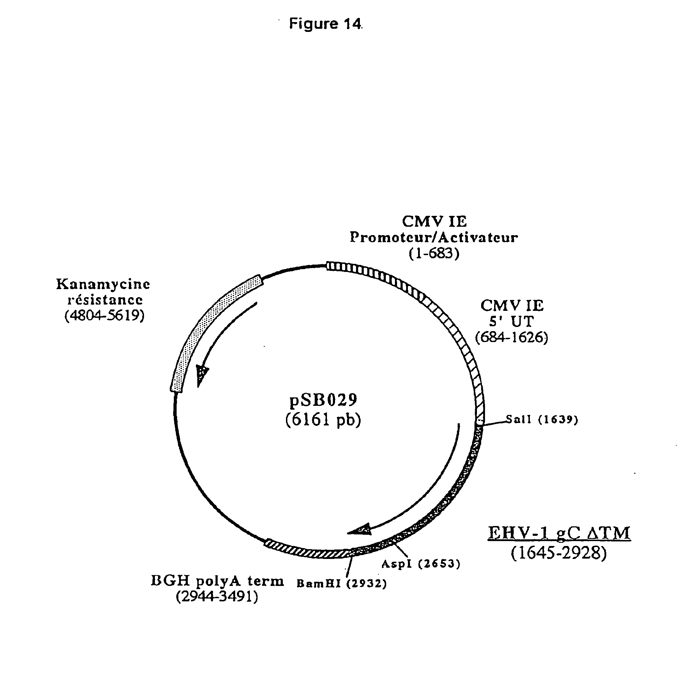

Figure N° 14 : Plasmide pSB029

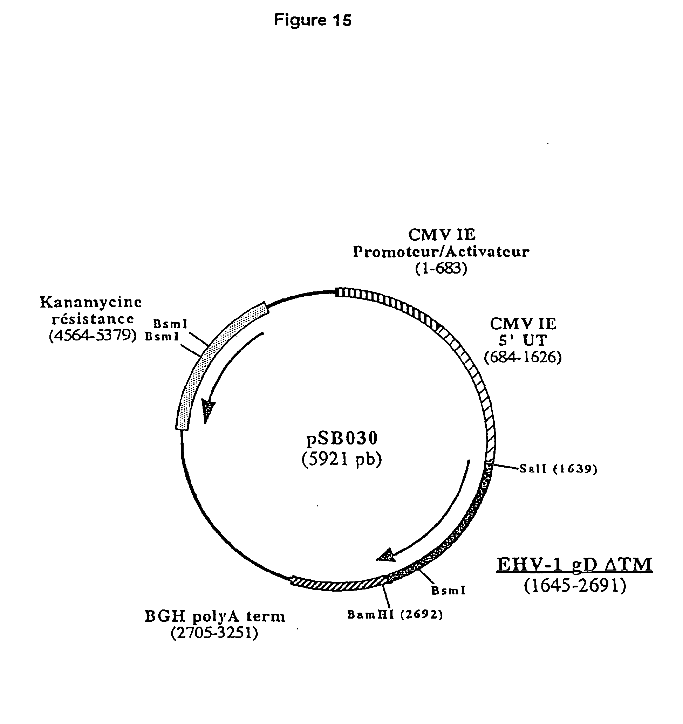

Figure N° 15 : Plasmide pSB030

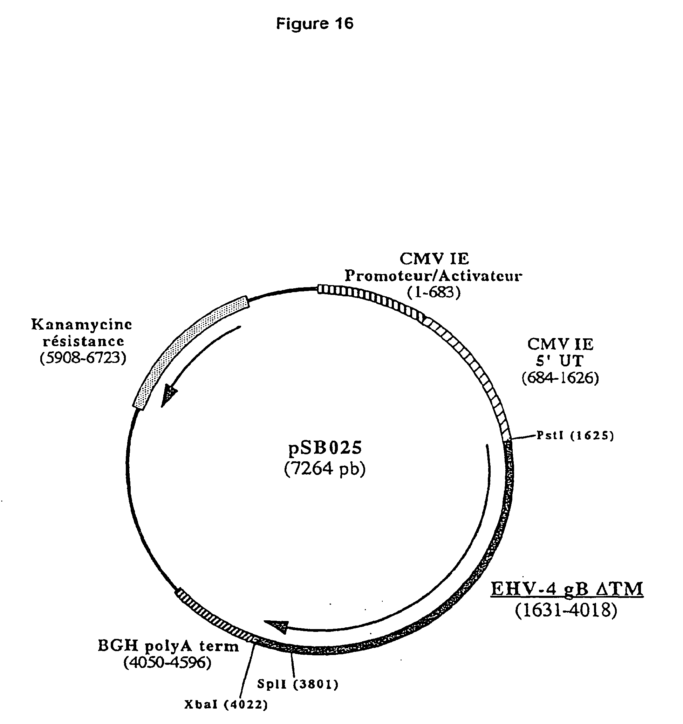

Figure N° 16 : Plasmide pSB025

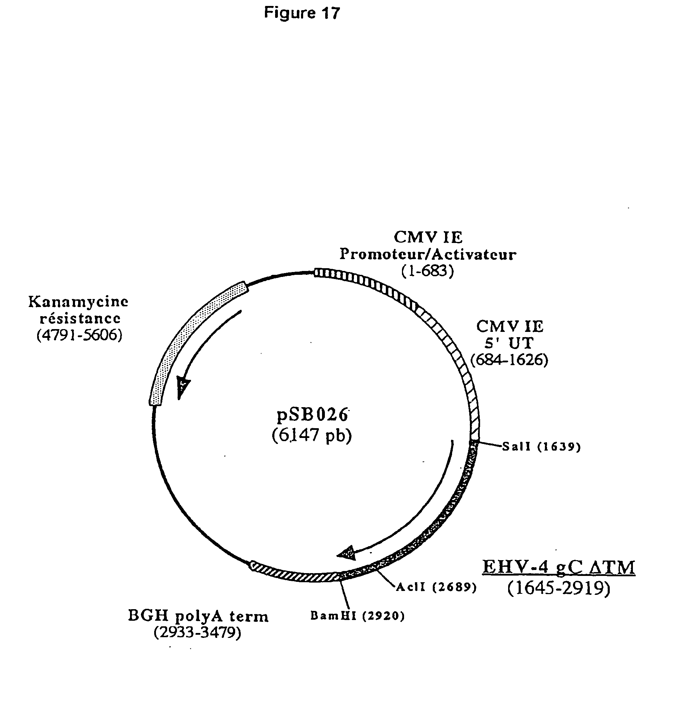

Figure N° 17 : Plasmide pSB026

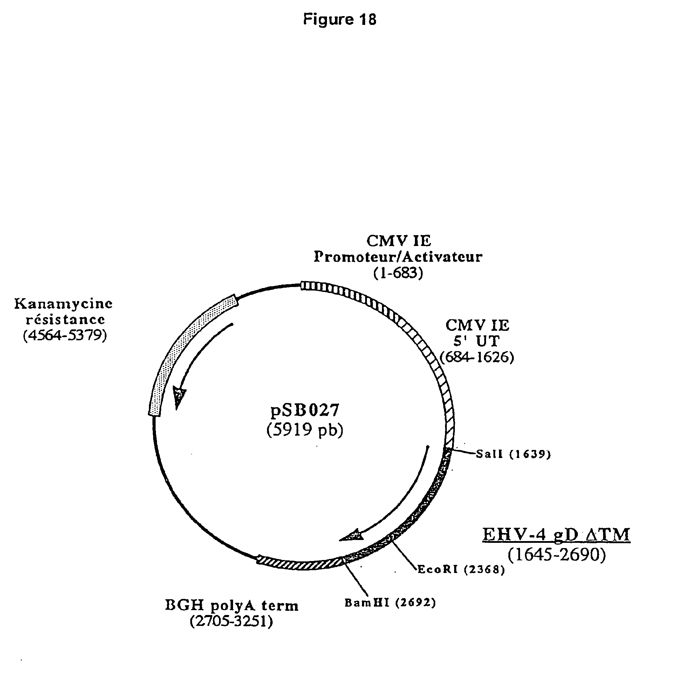

Figure N° 18 : Plasmide pSB027
Figure N° 19 : Plasmide pJP084
Figure N° 20 : séquence du gène GM-CSF canin
Figure N° 21 : Plasmide pJP089
Figure N° 22 : séquence du gène GM-CSF félin 3R3
Figure N° 23 : Plasmide pJP090
Figure N° 24 : séquence du gène GM-CSF félin 3R4
Figure N° 25 : Plasmide pJP097
Figure N° 26 : séquence du gène GM-CSF équin
Figure N° 27 : séquence du gène CPI-2 F
Figure N° 28 : séquence du gène CPI-2 HN

### Listes des séquences :

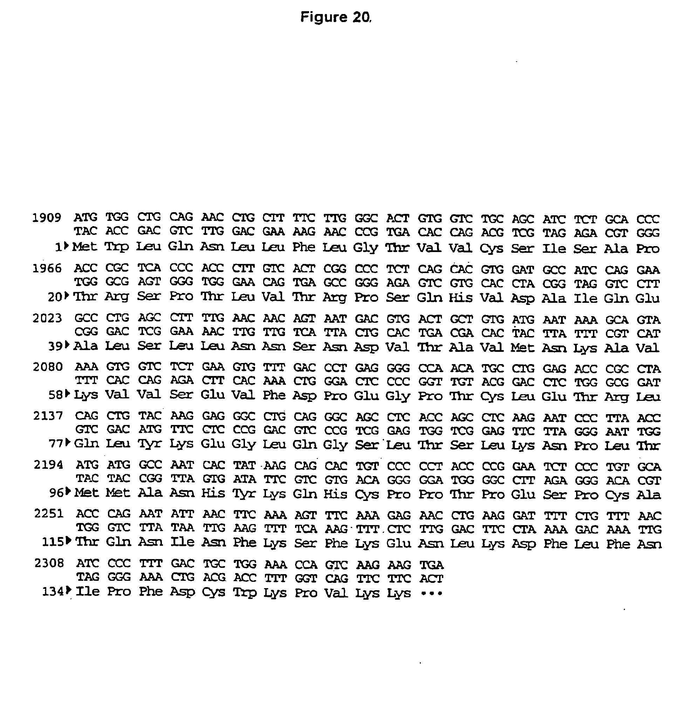

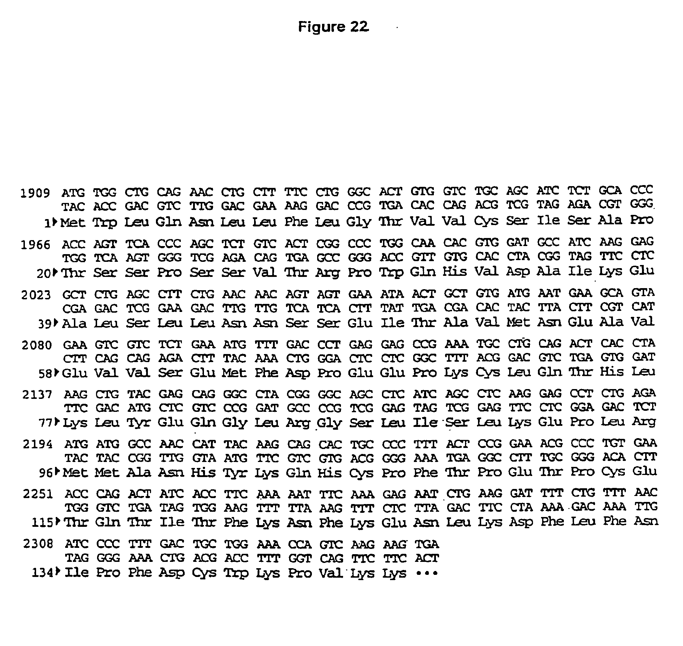

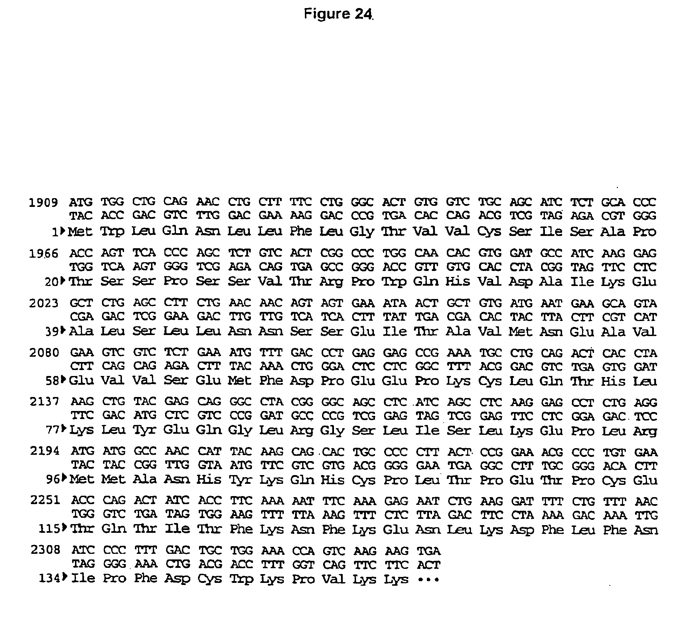

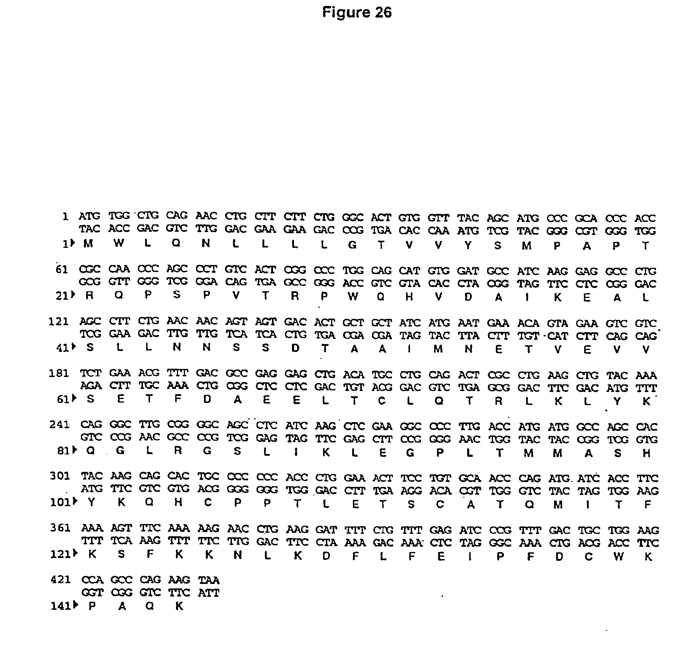

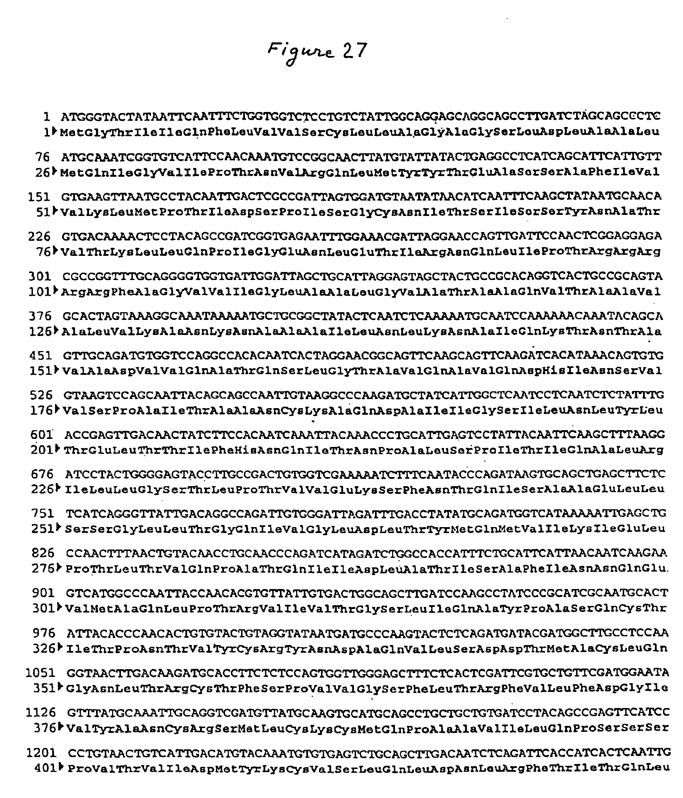

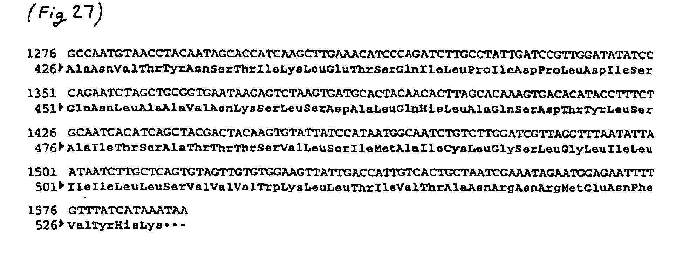

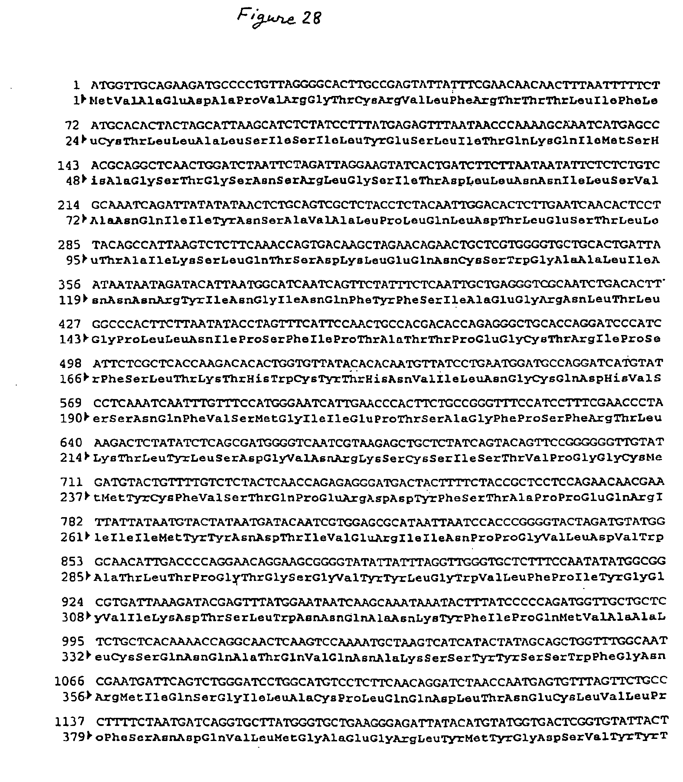

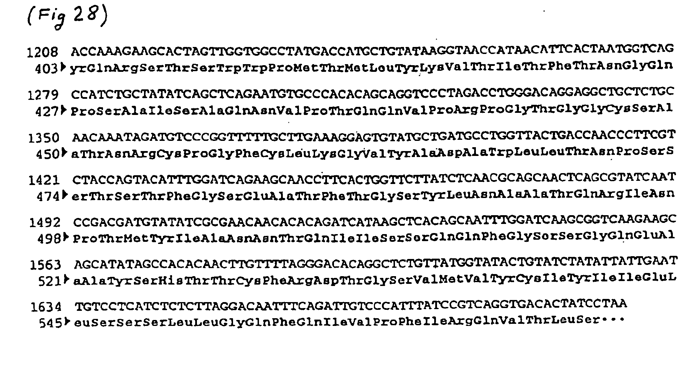

SET ID NO1 : oligonucléotide NS030
SEQ ID N° 2 : oligonucléotide NS031
SET ID N° 3 : oligonucléotide NS034
SEQ ID N° 4 : oligonucléotide NS035
SEQ ID N° 5 : oligonucléotide NS036
SEQ ID N° 6 : oligonucléotide NS037
SEQ ID N° 7 : oligonucléotide SB090
SEQ ID N° 8 : oligonucléotide SB091
SEQ ID N° 9 : oligonucléotide PB326
SEQ ID N° 10 : oligonucléotide PB329
SEQ ID N° 11 : oligonucléotide PB381
SEQ ID NO 12 : oligonucléotide PB382
SEQ ID NO 13 : oligonucléotide PB383
SEQ ID NO 14 : oligonucléotide PB384
SEQ ID NO 15 : oligonucléotide SB101
SEQ ID NO 16 : oligonucléotide SB102
SEQ ID NO 17 : oligonucléotide SB103
SEQ ID NO 18 : oligonucléotide SB104
SEQ ID NO 19 : oligonucléotide SB105
SEQ ID NO 20 : oligonucléotide SB106
SEQ ID NO 21 : oligonucléotide SB107
SEQ ID NO 22 : oligonucléotide SB108
SEQ ID NO 23 : oligonucléotide SB109
SEQ ID NO 24 : oligonucléotide SB110
SEQ ID NO 25 : oligonucléotide SB111
SEQ ID NO 26 : oligonucléotide SB112
SEQ ID NO 27 : oligonucléotide SB113
SEQ ID NO 28 : oligonucléotide SB114
SEQ ID NO 29 : oligonucléotide SB115
SEQ ID NO 30 : oligonucléotide SB116
SET ID NO 31 : oligonucléotide SB117
SEQ ID NO 32 : oligonucléotide SB118
SEQ ID NO 33 : oligonucléotide AB325
SEQ ID NO 34 : oligonucléotide AB326
SEQ ID NO 35 : oligonucléotide AB327
SEQ ID NO 36 : oligonucléotide AB328
SEQ ID NO 37 : oligonucléotide AB329
SEQ ID NO 38 : oligonucléotide AB330
SEQ ID NO 39 : oligonucléotide NS003
SEQ ID NO 40 : oligonucléotide NS004
SEQ ID NO 41 : oligonucléotide NS005
SET ID NO 42 : oligonucléotide NS006
SEQ ID NO 43 : oligonucléotide NS007
SEC ID NO 44 : oligonucléotide NS008
SEQ ID NO 45 : oligonucléotide SB119
SEC ID NO 46 : oligonucléotide SB120
SEQ ID NO 47 : oligonucléotide SB121
SEQ ID NO 48 : oligonucléotide SB122
SEQ ID NO 49 : oligonucléotide SB123
SEQ ID NO 50 : oligonucléotide SB124
SEQ ID NO 51 : oligonucléotide SB125
SET ID NO 52 : oligonucléotide SB126
SEQ ID NO 53 : oligonucléotide SB127
SEQ ID NO 54 : oligonucléotide SB128
SEQ ID NO 55 : oligonucléotide SB129
SEC ID NO 56 : oligonucléotide SB130
SEC ID NO 57 : oligonucléotide SB131
SEC ID NO 58 : oligonucléotide SB132
SEQ ID NO 59 : oligonucléotide SB133
SEC ID NO 60 : oligonucléotide SB134
SEQ ID NO 61 : oligonucléotide SB135
SEC ID NO 62 : oligonucléotide SB136
SEC ID NO 63 : oligonucléotide SB137
SEQ ID NO 64 : oligonucléotide JP578
SEQ ID NO 65 : oligonucléotide JP579
SEQ ID NO 66 : séquence du gène GM-CSF canin (voir figure 20)
SEQ ID NO 67 : séquence du gène GM-CSF félin 3R3 (voir figure 22)
SEQ ID NO 68 : séquence du gène GM-CSF félin 3R4 (voir figure 24)
SEQ ID NO 69 : séquence du gène GM-CSF équin (voir figure 26)
SEQ ID NO 70 : oligonucléotide JP734
SEQ ID NO 71 : oligonucléotide JP735
SEQ ID NO 72 : séquence du gène CPI-2 F (voir figure 27)
SEQ ID NO 73 : séquence du gène CPI-2 HN (voir figure 28)

### EXEMPLES.

Les exemples qui concernent le CDV sont illustratifs de l'invention. Les autres exemples sont donnés seulement pour information.

Pour les pathogènes considérés, chaque gène codant pour les principaux antigènes de surface (forme native et forme modifiée) a fait l'objet d'une construction particulière dans un plasmide d'expression eucaryote. Les formes secrétées des antigènes de surface ont été obtenues par délétion des fragments de gènes codant pour les domaines trans-membranaires et cytoplasmiques. Dans tous les cas, les domaines trans-membranaires des glycoprotéines ont été identifiés sur la base des profils d'hydropathie des séquences protéiques correspondantes. Le tableau donné dans l'exemple 11 résume les tailles des protéines sauvages (en acides aminés), les positions identifiées des domaines trans-membranaires, les tailles des protéines tronquées, ainsi que les désignations des plasmides d'expression correspondants.

### Exemple 1 : Constructions plasmidiques de base

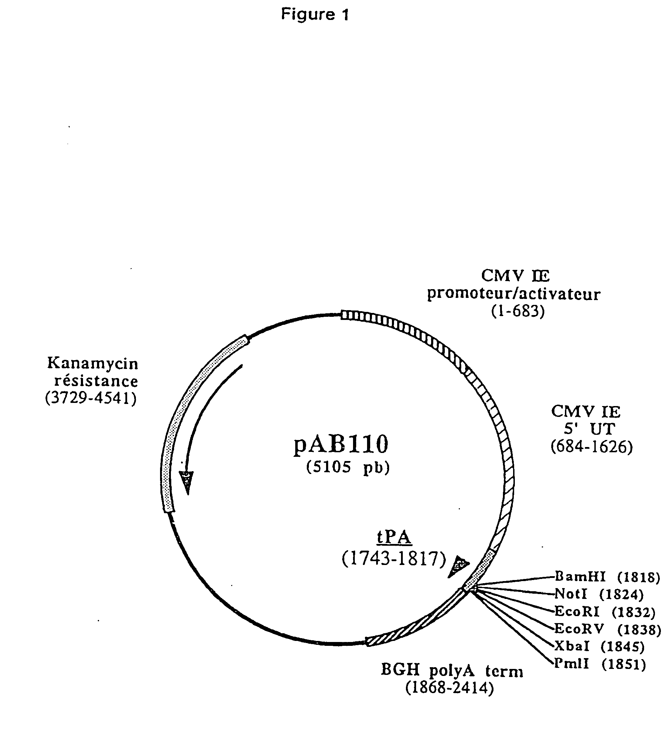

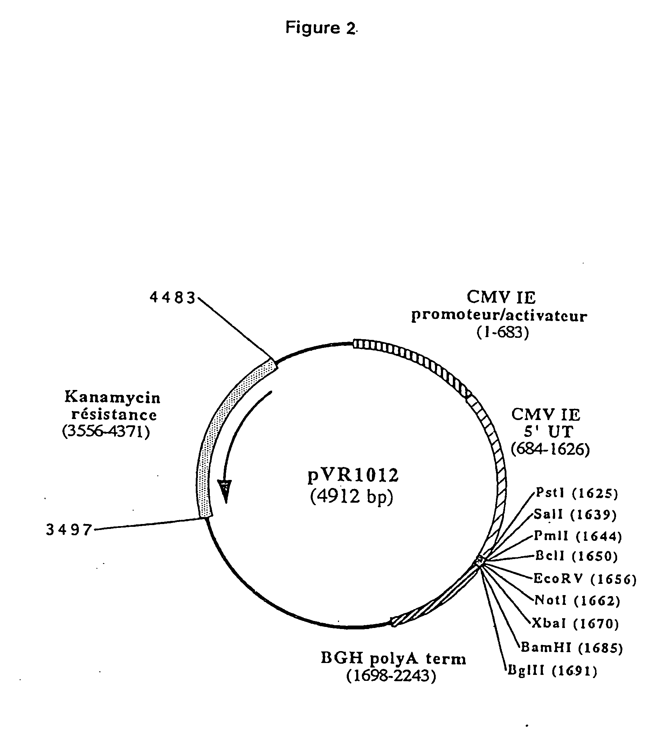

Le plasmide d'expression eucaryote pVR1020 (C. J. Luke et al. J. of Infectious Diseases 1997, 175 : 95-97), dérivé du plasmide pVR1012 (figure 1, exemple 7 de WO-A-9803199, repris en figure 2 de la présente demande), contient la phase codante de la séquence-signal de l'activateur du plasminogène tissulaire humain (tPA). Le plasmide pVR1020 a été modifié par digestion BamHI-BgIII et insertion d'une séquence contenant plusieurs sites de clonage

(BamHI, NotI, EcoRI, Xbal, PmII, PstI, BgIII) et résultant de l'appariement des oligonucléotides suivants :
PB326 (40 mer) (SEQ ID NO 9)
   5' GATCTGCAGCACGTGTCTAGAGGATATCGAATTCGCGGCC 3' et
PB329 (40 mer) (SEQ ID NO 10)
   5' GATCCGCGGCCGCGAATTCGATATCCTCTAGACACGTGCT 3'.

Le vecteur résultant, pAB110 (Figure N° 1), a été utilisé pour la construction des plasmides contenant les formes tronquées des gènes codant pour l'hémagglutinine (HA) du virus de la maladie de Carré (CDV) et pour l'hémagglutinine-neuraminidase (HN) du virus parainfluenza de type 2 (CPI-2).

L'intron II du gène de la β-globine de lapin a été cloné dans le vecteur pCRII (Invitrogen, Carlsbad, CA, USA) après obtention du fragment d'ADN correspondant par PCR à l'aide des oligonucléotides suivants :
SB090 (20 mer) (SEQ ID NO 7)
   5' TTGGGGACCCTTGATTGTTC 3' et
SB091 (21 mer) (SEQ ID NO 8)
   5' CTGTAGGAAAAAGAAGAAGGC 3'
en utilisant comme matrice l'ADN génomique de cellules du sang périphérique de lapin. Le plasmide résultant a été désigné pNS050.

### Exemple 2 : Plasmides codant pour les différentes formes des antigènes de CDV

Les gènes codant pour la protéine de fusion (F) et l'hémagglutinine (HA) de CDV souche Snyder Hill (SH) ont été obtenus par RT-PCR à partir de l'ARN viral de la souche SH (accessible auprès de la souchothèque American Tissue Culture Collection sous le numéro ATCC VR-526).

### 2.1. Plasmides codant pour les différentes formes de CDV-F

### 2.1.1. pPB229: gène F (forme native) cloné dans le vecteur pVR1012

L'ADNc du gène F de CDV a été synthétisé à l'aide de l'amorce PB383 et amplifié par une réaction de PCR à l'aide du couple d'oligonucléotides suivant :
PB383 (26 mer) (SEQ ID NO 13)
   5' TTTCTAGACAGCCGAGCCCCATGCAC 3' et
PB384 (30 mer) (SEQ ID NO 14)
   5' TTGGATCCGATATATGACCAGAATACTTCA 3'.
Le produit de PCR a été digéré par BamHI et Xbal et cloné dans le vecteur d'expression pVR1012 (exemple 1) préalablement digéré par BamHI et Xbal, générant le plasmide pPB229 (6925 paires de bases ou pb). Le gène F sauvage de CDV code pour une protéine de 662 résidus.

### 2.1.2. pNS021: gène F (forme β-globine F ΔTM) cloné dans le vecteur pVR1012

Le plasmide pNS013 (6735 pb) contenant le gène F tronqué du domaine trans-membranaire et C-terminal a été obtenu par la ligation d'un fragment Bsu36I-BamHI (6593 pb) issu du plasmide pPB229 (exemple 2.1.1), et d'un fragment de 142 pb obtenu par PCR à partir de la matrice pPB229 à l'aide des oligonucléotides suivants :
NS030 (21 mer) (SEQ ID NO 1)
   5' ATGAGCCCACTCTTACAACAA 3' et
NS031 (35 mer) (SEQ ID NO 2)
   5' TTTCGCGGATCCATTAAAGGAAGAGCGCCTAACCG 3'
et digéré Bsu36I-BamHI. Le gène F troqué de CDV code pour une protéine de 605 résidus.

Dans un deuxième temps, une séquence correspondant à l'intron II du gène de la β-globine du lapin a été insérée en amont de la séquence codante du gène F tronqué, dans le site SalI du plasmide pNS013. Le fragment d'ADN correspondant à l'intron (573 pb) a été obtenu par PCR à l'aide des oligonucléotides suivants :
NS036 (34 mer) (SEQ ID NO 5)
   5' TTTACGCGTCGACTTGGGGACCCTTGATTGTTC 3' et
NS037 (36 mer) (SEQ ID NO 6)
   5' TTTACGCGTCGACCTGTAGGAAAAAGAAGAAGGCAT 3'
sur la matrice pNS050 (exemple 1), suivie par une digestion SaII pour libérer des extrémités compatibles SalI. Le dérivé du plasmide pNS013 contenant l'intron II du gène de la β-globine est désigné pNS021 (7308 pb) (Figure N°3).

### 2.2. Plasmides codant pour les différentes formes de CDV-HA

### 2.2.1, pNS018: gène HA (forme native) cloné dans le vecteur pVR1012

L'ADNc du gène HA de CDV a été synthétisé à l'aide de l'amorce PB381 et amplifié par réaction PCR à l'aide du couple d'oligonucléotides suivant :
PB381 (30 mer) (SEQ ID NO 11)
   5' TTCTGCAGATGCTCTCCTACCAAGAYAAGG 3' et
PB382 (28 mer) (SEQ ID NO 12)
   5' TTGTCGACATGTGTATCATCATMCTGTC 3'.

Le produit PCR a été cloné dans le vecteur pCRII (Invitrogen, Carlsbad, CA, USA), générant le plasmide pPB235. Le fragment PstI-SaII de 1846 pb du plasmide pPB235 contenant le gène HA a été ensuite cloné dans le vecteur d'expression pVR1012 (exemple 1) digéré PstI-SaII, générant le plasmide pNS018 (6748 pb). Le gène HA sauvage de CDV code pour une protéine de 607 résidus.

### 2.2.2. pNS024: gène HA (forme β-globine tPA ΔTM HA) cloné dans le vecteur pVR1012

La forme tronquée du gène HA de CDV a été obtenue par délétion du fragment d'ADN codant pour les 60 premiers résidus de la protéine HA. La séquencesignal et trans-membranaire de cette protéine étant confondues, la secrétion du produit tronqué est assurée par l'obtention d'une fusion en phase entre la séquence signal de l'activateur du plasminogène tissulaire humain (tPA) et le gène HA tronqué. Le plasmide pNS019, le dérivé de pNS018 codant pour un produit de fusion avec le signal tPA, a été obtenu par la ligation des 3 fragments d'ADN suivants:
- Le fragment A a été obtenu par digestion BamHI-EcoRV de pAB110 (exemple 1).
- Le fragment B a été obtenu par réaction PCR sur la matrice pNS018 (exemple 2.2.1) à l'aide des oligonucléotides suivants :
   NS034 (30 mer) (SEQ ID NO 3)
      5' TTTCGCGGATCCCACAAAGTATCAACTAGC 3' et
   NS035 (23 mer) (SEQ ID NO 4)
      5' GGGATTTGCTGCCGATGCAATAG 3'
   suivie par une digestion BamHI-SapI du produit PCR.
- Le fragment C est un fragment de digestion SapI-EcoRV de pNS018.

Le gène hybride tPA ΔTM HA code pour une protéine de 574 résidus (1725 pb).

L'intron Il du gène de la β-globine de lapin (exemple 2.1.2) a été inséré en amont de la phase codante du gène HA dans le site SaII de pNS019 pour générer le plasmide pNS024 (Figure N° 4).

### Exemple 3 : Plasmides codant pour les différentes formes des antigènes du virus parainfluenza canin de type 2 (CPI-2)

Les gènes F et HN de CPI-2 souche D008 (MERIAL) ont été obtenus par RT-PCR à partir de l'ARN viral.

### 3.1. Plasmides codant pour les différentes formes de CPI-2 F

### 3.1.1. pAB115: gène F (forme native) cloné dans le vecteur pVR1012

L'ADNc du gène F de CPI-2 a été synthétisé et amplifié par RT-PCR à l'aide du couple d'oligonucléotides suivant :
SB131 (38 mer) (SEQ ID NO 57)
   5' AAAAACGCGTCGACATGGGTACTATAATTCAATTTCTG 3'
SB132 (38 mer) (SEQ ID NO 58)
   5' TTTTCTAGTCTAGATTATTTATGATAAACAAAATTCTC 3'.

Le produit de PCR a été digéré par SalI et XbaI, générant un fragment de 1594 pb, et cloné dans le vecteur d'expression pVR1012 (exemple 1) préalablement digéré par les mêmes enzymes, générant le plasmide pAB115 (6479 pb). Le gène F sauvage de CPI-2 (SEQ ID NO72) (figure 27) cloné sur ce plasmide code pour une protéine de 529 résidus.

### 3.1.2. pSB032: gène F (forme β-globine F ΔTM) cloné dans le vecteur pVR1012

Le plasmide pSB031 contenant le gène F tronqué des domaines trans-membranaire et cytoplasmique C-terminal été obtenu de la façon suivante. Une réaction PCR a été réalisée avec la matrice pAB115 (exemple 3.1.1.) à l'aide des oligonucléotides suivants :
SB131 (SEQ ID NO 57) et
SB133 (41 mer) (SEQ ID NO 59)
   5' TTTTCTAGTCTAGATTAGTATGTGTCACTTTGTGCTAAGTG 3'
pour générer un fragment PCR d'environ 1450 pb. Ce fragment a été digéré par par SaII et XbaI pour isoler le fragment de restriction SaII-XbaI de 1436 pb. Ce fragment a été ligaturé avec le vecteur pVR1012 (exemple 1) préalablement digéré par SaII et XbaI pour donner le plasmide pSB031. Le gène F tronqué de CPI-2 code pour une protéine de 473 résidus.

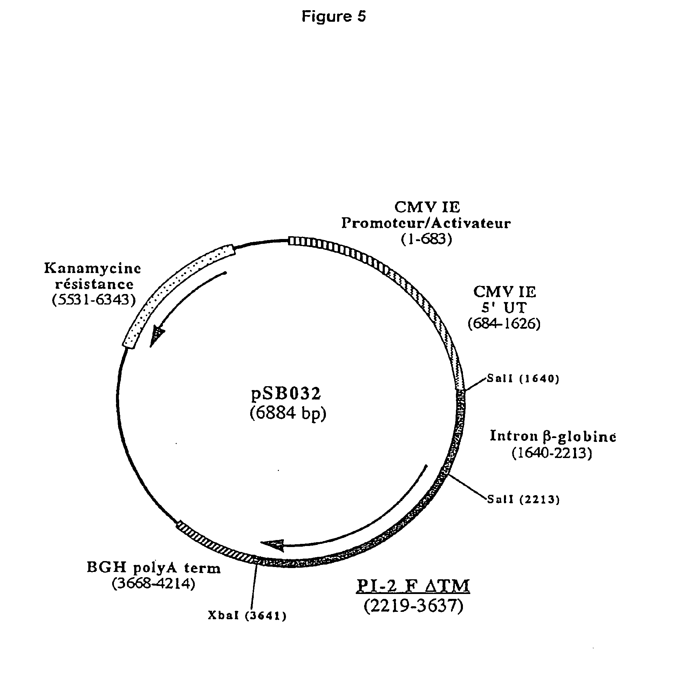

Dans un deuxième temps, une séquence correspondant à l'intron II du gène de la β-globine du lapin a été insérée en amont de la séquence codante du gène F tronqué de CPI-2, dans le site SaII du plasmide pSB031. Le fragment d'ADN correspondant à l'intron (573 pb) a été obtenu par PCR à l'aide des oligonucléotides NS036 (SEQ ID NO 5) et NS037 (SEQ ID NO 6) sur la matrice pNS050 (exemple 1), suivie par une digestion SaII pour libérer des extrémités compatibles SaII. Ce fragment de restriction SaII-SaII a été ligaturé avec le plasmide pSB031 préalablement digéré par SaII, puis déphosphorylé pour donner le plasmide pSB032 (6884 pb) (Figure N° 5).

### 3.2. Plasmides codant pour les différentes formes de CPI-2 HN

### 3.2.1. pAB114: gène HN (forme native) cloné dans le vecteur pVR1012

L'ADNc du gène HN de CPI-2 a été synthétisé et amplifié l'aide des oligonucléotides suivants :
SB134 (41 mer) (SEQ ID NO 60)
   5' AAAAACGCGTCGACATGGTTGCAGAAGATGCCCCTGTTAGG 3'
SB135 (35 mer) (SEQ ID NO 61)
   5' TTTTGGAAGATCTTTAGGATAGTGTCACCTGACGG 3'
pour générer un fragment PCR d'environ 1720 pb. Ce fragment a été digéré par SaII et BgIII pour isoler le fragment SaII-BgII de 1704 pb. Ce fragment a alors été ligaturé avec le vecteur pVR1012 (exemple 1), préalablement digéré par Sali et BgIII, pour donner le plasmide pAB114 (6566 pb). Le gène HN sauvage de CPI-2 (SEQ ID NO73) (figure 28) cloné sur ce plasmide code pour une protéine de 565 résidus.

### 3.2.2. pSB034: gène HN (forme β-globine HN tPA ΔTM) cloné dans le vecteur pVR1012

La forme tronquée du gène HN de CPI-2 a été obtenue par délétion du fragment d'ADN codant pour les 40 premiers résidus de la protéine HN. Les séquences signal et trans-membranaire de cette protéine étant confondues, la secrétion du produit tronqué est assurée par l'obtention d'une fusion en phase entre la séquence signal de l'activateur du plasminogène tissulaire humain (tPA) et le gène HN tronqué. Le plasmide pSB033, dérivé de pAB114 (exemple 3.2.1.), codant pour un produit de fusion avec le tPA, a été obtenu par la ligation du fragment EcoRI-PmII de pAB110 (exemple 1), un dérivé de pVR1012 contenant une phase ouverte de lecture codant pour la séquence signal tPA et d'un fragment de (1599 pb) obtenu par PCR à l'aide des oligonucléotides suivants :
SB136 (37 mer) (SEQ ID NO 62)
   5'TTAAAAGAATTCGACCCAAAAGCAAATCATGAGCCAC 3'
SB137 (33 mer) (SEQ ID NO 63)
   5' TTAAAAGGCCTTTAGGATAGTGTCACCTGACGG 3'
sur la matrice pAB114 et digéré par EcoRI et EcoRV.

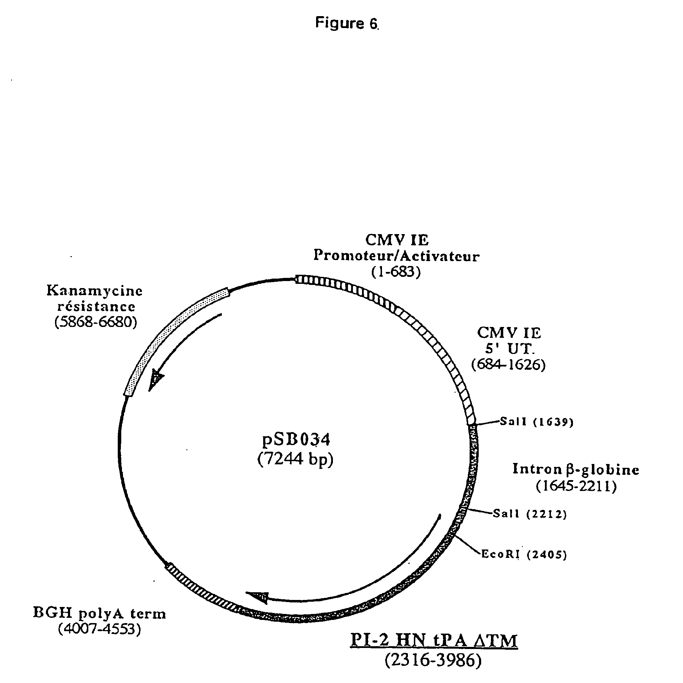

L'intron II du gène de la β-globine de lapin a été inséré en amont de la phase codante du gène HN dans le site SaII de pBS033 pour générer le plasmide pSB034 (Figure N° 6). Le fragment SaII contenant l'intron a été obtenu par PCR à l'aide des oligonucléotides NS036 (SEQ ID NO 5) et NS037 (SEQ ID NO 6) sur la matrice pNS050 (exemple 1).

### Exemple 4: Plasmides codant pour les différentes formes des glycoprotéines du virus CHV-1

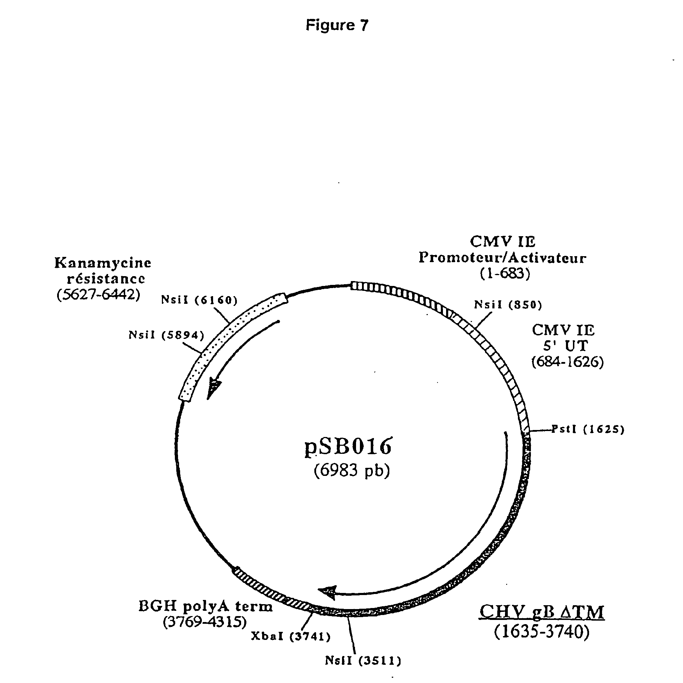

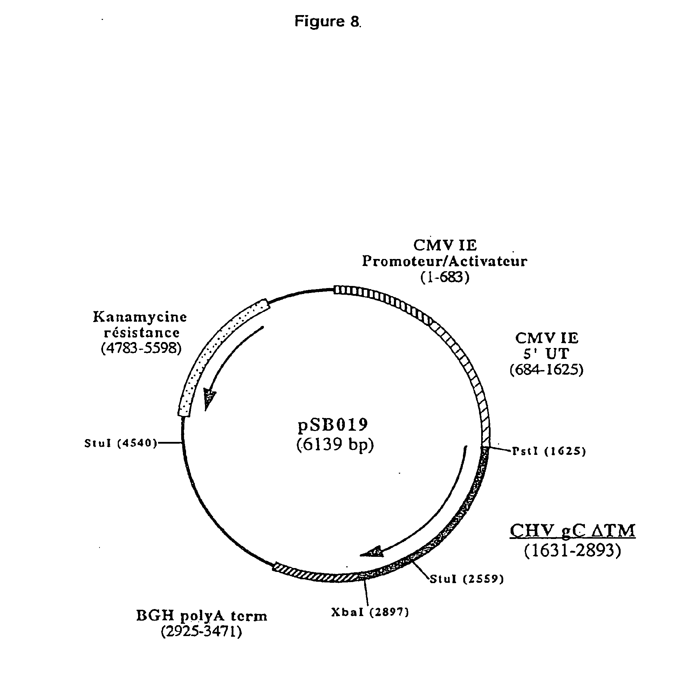

Les gènes codant pour les glycoprotéines gB, gC et gD de la souche Carmichael du virus herpès canin de type 1 (CHV-1) ont été isolés par PCR à partir du génome viral. Le clonage des gènes codant pour gB et gD dans le vecteur pVR1012 a été précédemment décrit dans la demande de brevet WO-A-9803199 (plasmides pAB037 et pAB038, respectivement aux figures 7 et 8 et aux exemples 13 et 14). En revanche, le clonage du gène codant pour gC est décrit dans ce document.

### 4.1. Plasmide codant pour la forme tronquée de CHV-gB

### 4.1.1. pSB016: gène gB (forme ΔTM) cloné dans le vecteur pVR1012

Selon le profil d'hydropathie, le domaine trans-membranaire de la protéine gB de CHV-1 (878 acides aminés) est positionné entre les résidus 702 et 769. Le plasmide contenant la forme tronquée du gène codant pour gB a été obtenu par la ligature des trois fragments d'ADN suivants : (a) le vecteur pVR1012 (exemple 1) linéarisé par une double digestion Pstl-Xbal, (b) un fragment de 1997 pb obtenu par la digestion Pstl-Nsil de pAB037 (exemple 4) et (c) un fragment de 225 pb obtenu par PCR à l'aide des oligonucléotides suivants :
SB101 (22 mer) (SEQ ID NO 15)
   5'TATATTGAAGGACAACTTGGGG 3'et
SB102 (36 mer) (SEQ ID NO 16)
   5'CTAGTCTAGATTAATTATTATCAACTTTTACAACAC 3'
en utilisant le plasmide pAB037 comme matrice et digéré par Nsil et Xbal. Le plasmide résultant, pSB016 (6983 pb) (Figure N° 7) contient un gène gB tronqué codant pour une protéine de 701 résidus.

### 4.2. Plasmides codant pour les différentes formes de CHV-gC

### 4.2.1. pSB018: gène gC (forme native) cloné dans le vecteur pVR1012

Le fragment d'ADN contenant la phase ouverte de lecture du gène gC de CHV-1 a été obtenu par PCR à l'aide des oligonucléotides suivants :
SB105 (32 mer) (SEQ ID NO 19)
   5'AAAACTGCAGATGAGTTTTAAAAATTTTTATC 3' et
SB106 (30 mer) (SEQ ID NO 20)
   5'CTAGTCTAGATTAGATCTTATTATTTTTTG 3'
en utilisant l'ADN viral comme matrice. Ce produit PCR a été digéré par Pstl et Xbal générant un fragment de 1400 pb, qui a ensuite été ligaturé dans le vecteur pVR1012 (exemple 1) linéarisé par la même double digestion. Le plasmide résultant, pSB018 (6253 pb) contient le gène codant pour la glycoprotéine gC de 459 résidus.

### 4.2.2. pSB019: gène gC (forme ΔTM) cloné dans le vecteur pVR1012

Selon le profil d'hydropathie, le domaine trans-membranaire de la protéine gC est compris entre les résidus 422 et 452. Le plasmide contenant la forme tronquée du gène codant pour gD a été obtenu par la ligature des trois fragments d'ADN suivants : (a) le vecteur pVR1012 (exemple 1) linéarisé par une double digestion Pstl-Xbal, (b) un fragment de 934 pb obtenu par la digestion Pstl-Stul de pSB018 (exemple précédent) et (c) un fragment de 335 pb obtenu par PCR à l'aide des oligonucléotides suivants :
SB107 (24 mer) (SEQ ID NO 21)
   5'TGGATTGACGGTCTTATAACACGC 3' et
SB108 (37 mer) (SEQ ID NO 22)
   5'CTAGTCTAGATTAATTTTCATCCGATGCATCAAACAC 3'
en utilisant le plasmide pSB018 comme matrice et digéré par StuI et XbaI. Le plasmide résultant, pSB019 (6139 pb) (Figure N° 8), contient un gène gC tronqué codant pour une protéine de 421 résidus.

### 4.3. Plasmide codant pour la forme tronquée de CHV-gO

### 4.3.1 pSB017: gène gD (forme ΔTM) cloné dans le vecteur pVR1012

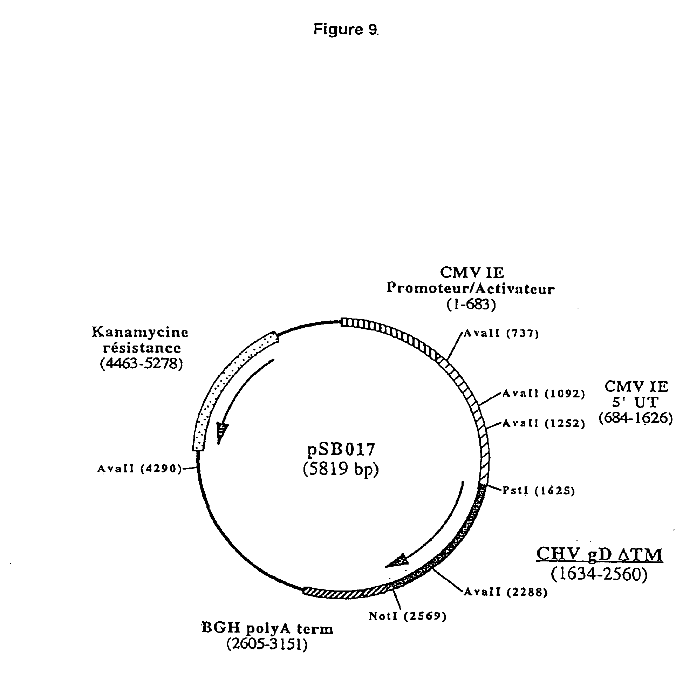

Le domaine trans-membranaire de la protéine gD de CHV-1 (345 acides aminés) est compris entre les résidus 310 et 328. Le plasmide contenant la forme tronquée du gène codant pour gD a été obtenu par la ligature des trois fragments d'ADN suivants : (a) le vecteur pVR1012 (exemple 1) linéarisé par une double digestion Pstl-Notl, (b) un fragment de 663 pb obtenu par la digestion Pstl-Avall de pAB038 (exemple 4) et (c) un fragment de 415 pb obtenu par PCR à l'aide des oligonucléotides suivants :
SB103 (25 mer) (SEQ ID NO 17)
   5'CGAGAAACTTGTTATTTTTCTAAAG 3' et
SB104 (51 mer) (SEQ ID NO 18)
   5'ATAAGAATGCGGCCGCAAAGGCTATATATTTTTTGGGGTATTATTTATTGG 3'
en utilisant le plasmide pAB038 comme matrice et digéré par AvaII et NotI. Le plasmide résultant, pSB017 (5819 pb) (Figure N° 9), contient un gène gD tronqué codant pour une protéine de 309 résidus.

### Exemple 5 : Plasmides codant pour les différentes formes des glycoprotéines de FHV-1

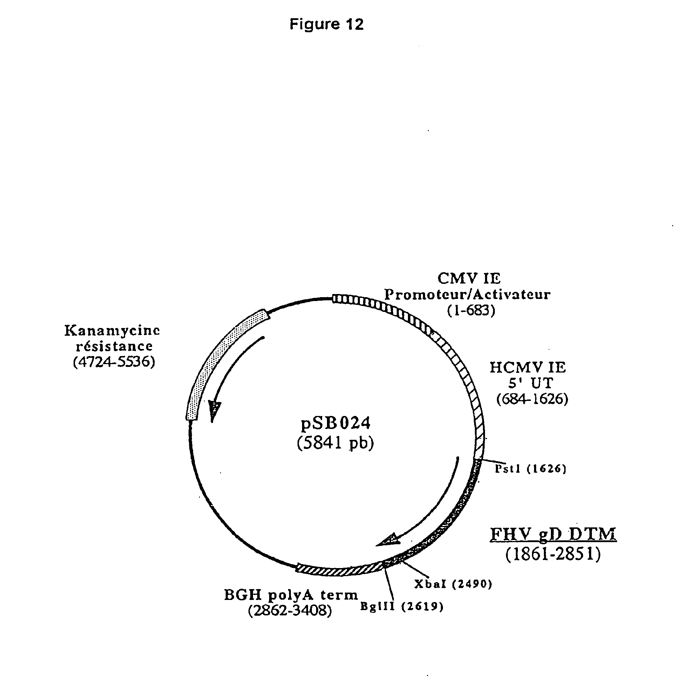

Les gènes codant pour les glycoprotéines gB, gC et gD de la souche CO du virus herpès félin de type 1 (FHV-1) ont été isolés par PCR à partir du génome viral. Dans le cas particulier du gène codant pour gD, dont la séquence nucléotidique est identique à celle de la souche C-27, nous avons utilisé le plasmide pAB029, dérivé de pVR1012 contenant le gène correspondant cloné à partir de la souche C-27 et décrit dans la demande de brevet WO-A-9803660 (plasmide pAB029, figure 12 et exemple 15).

### 5.1. Plasmides codant pour les différentes formes de FHV-gB

### 5.1.1. pSB020: gène gB (forme native) cloné dans le vecteur pVR1012

Le fragment d'ADN contenant la phase ouverte de lecture du gène gB de FHV-1 a été obtenu par PCR à l'aide des oligonucléotides suivants :
SB113 (34 mer) (SEQ ID NO 27).
   5'TTTTCTGCAGATGTCCACTCGTGGCGATCTTGGG 3' et
SB114 (40 mer) (SEQ ID NO 28)
   5'ATAGTTTAGCGGCCGCTTAGACAAGATTTGTTTCAGTATC 3'
en utilisant l'ADN viral comme matrice. Le produit PCR a été digéré par Pstl et NotI, générant un fragment de 2849 pb, qui a ensuite été ligaturé dans le vecteur pVR1012 (exemple 1) linéarisé par la même double digestion. Le plasmide résultant, pSB020 (7728 pb), contient le gène codant pour la glycoprotéine gB de 949 résidus.

### 5.1.2. pSB021: gène gB (forme ΔTM) cloné dans le vecteur pVR1012

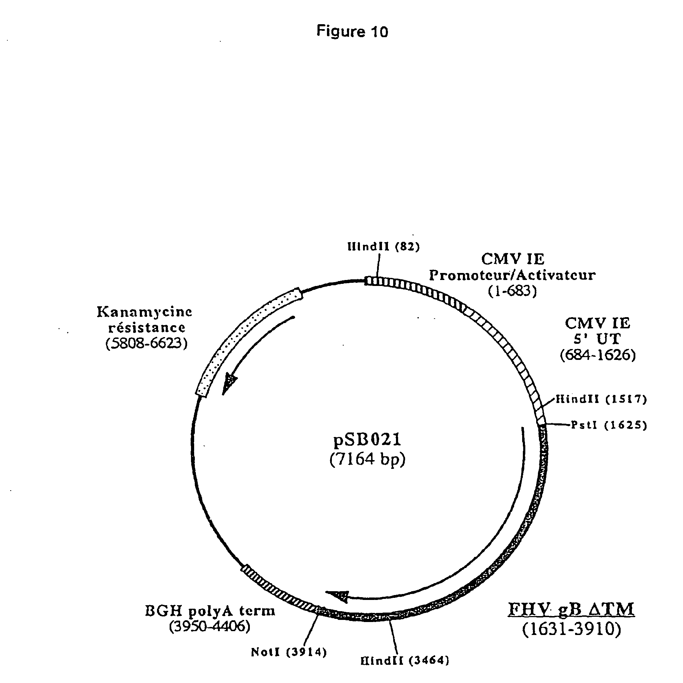

Selon le profil d'hydropathie, le domaine trans-membranaire de la protéine gB de FHV-1 est situé entre les résidus 761 et 834. Le plasmide contenant la forme tronquée du gène codant pour gB a été obtenu par la ligature des trois fragments d'ADN suivants : (a) le vecteur pVR1012 (exemple 1) linéarisé par une double digestion PstI-NotI, (b) un fragment de 1839 pb obtenu par la digestion Pstl-Hindll de pSB020 (exemple précédent) et (c) un fragment de 447 pb obtenu par PCR à l'aide des oligonucléotides suivants :
SB109 (24 mer) (SEQ ID NO 23)
   5' CTGTGGACAGAGACCCTAAAACTC 3' et
SB110 (50 mer) (SEQ ID NO 24)
   5' TTTCCTTTTGCGGCCGCTTATATGCTGTCTATATCATAAAATTTTAAGGC 3'
en utilisant le plasmide pSB020 comme matrice et digéré par HindII et NotI. Le plasmide résultant, pSB021 (7164 pb) (Figure N° 10), contient un gène gB tronqué codant pour une protéine de 760 résidus.

### 5.2. Plasmides codant pour les différentes formes de FHV-gC

### 5.2.1. pSB022: gène gC (forme native) cloné dans le vecteur pVR1012

Le fragment d'ADN contenant la phase ouverte de lecture du gène gC de FHV-1 a été obtenu par PCR à l'aide des oligonucléotides suivants :
SB115 (34 mer) (SEQ ID NO 29)
   5'TTTTCTGCAGATGAGACGATATAGGATGGGACGC 3' et
SB116 (34 mer) (SEQ ID NO 30)
   5'AGTTTAGCGGCCGCTTATAATCGCCGGGGATGAG 3'
en utilisant l'ADN viral comme matrice. Ce produit PCR a été digéré par PstI et Notl, générant un fragment de 1605 pb, qui a ensuite été ligaturé dans le vecteur pVR1012 (exemple 1) linéarisé par la même double digestion. Le plasmide résultant, pSB022 (6483 pb) contient le gène codant pour la glycoprotéine gC de 534 résidus.

### 5.2.2. pSB023: gène gC (forme ΔTM) cloné dans le vecteur pVR1012

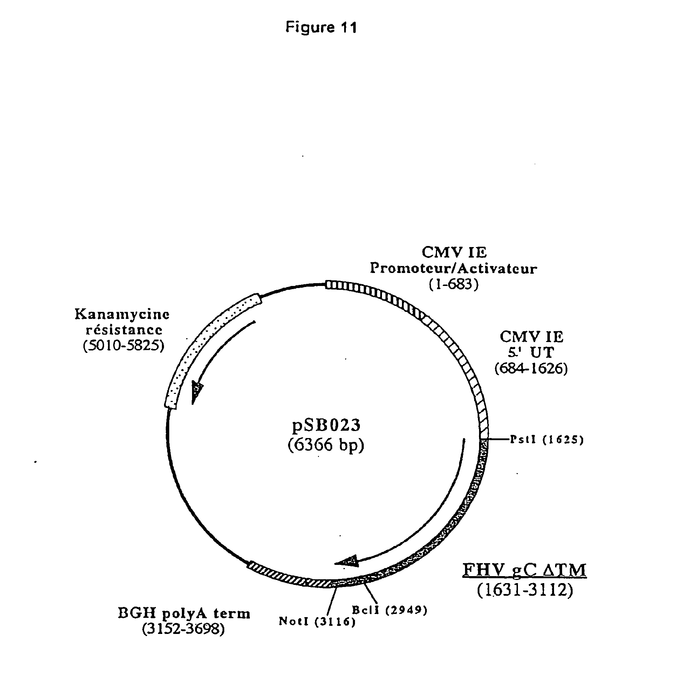

Selon le profil d'hydropathie, le domaine trans-membranaire de la protéine gC de FHV-1 est compris entre les résidus 495 et 526. Le plasmide contenant la forme tronquée du gène codant pour gC a été obtenu par la ligature des deux fragments d'ADN suivants : (a) un fragment de 6198 pb obtenu par la digestion Bcll-Notl de pSB022 (exemple précédent) et (b) un fragment de 168 pb obtenu par PCR à l'aide des oligonucléotides suivants :
SB117 (24 mer) (SEQ ID NO 31)
   5'GTTAAATGTGTACCACGGGACGGG 3' et
SB118 (41 mer) (SEQ ID NO 32)
   5'AGTTTAGCGGCCGCTTATTCAGGGGACGCGTCGTAGACTTG 3'
en utilisant le plasmide pSB022 comme matrice et digéré par BcII et NotI. Le plasmide résultant, pSB023 (6366 pb) (Figure N° 11), contient un gène gC tronqué codant pour une protéine de 494 résidus.

### 5.3. Plasmide codant pour la forme tronquée de FHV-gD

### 5.3.1. pSB024: gène gD (forme ΔTM) cloné dans le vecteur pVR1012

Le domaine trans-membranaire de la protéine gD de FHV-1 (374 acides aminés) est compris entre les résidus 328 et 353. Le plasmide contenant la forme tronquée du gène codant pour gD a été obtenu par la ligature des deux fragments d'ADN suivants : (a) un fragment de 5712 pb obtenu par la digestion XbaI-BglII de pAB029 (exemple 5) et (b) un fragment de 129 pb obtenu par PCR à l'aide des oligonucléotides suivants :
SB111 (24 mer) (SEQ ID NO 25)
   5'GATCGTCCCGCCATACCGTCTGGG 3' et
SB112 (39 mer) (SEQ ID NO 26)
   5'TTTGGAAGATCTTTACTGATTATTCATGCCCTTGGGAGG 3'
en utilisant le plasmide pAB029 comme matrice et digéré par XbaI et BglII. Le plasmide résultant, pSB024 (5841 pb) (Figure N° 12), contient un gène gD tronqué codant pour une protéine de 327 résidus.

### Exemple 6: Plasmides codant pour les différentes formes des glycoprotéines de EHV-1

Les gènes codant pour les glycoprotéines gB, gC et gD de la souche 2234/88-2 de EHV-1 ont été isolés par PCR à partir de l'ADN viral purifié.

### 6.1. Plasmides codant pour les différentes formes de EHV-1 gB

### 6.1.1. pAB127: gène gB (forme native) cloné dans le vecteur pVR1012

La phase codante du gène gB de EHV-1 a été amplifiée par PCR à l'aide des oligonucléotides suivants :
NS003 (30 mer) (SEQ ID NO 39)
   5' TTCTGCAGATGTCCTCTGGTTGCCGTTCGT 3'et
NS004 (30 mer) (SEQ ID NO 40)
   5' TTTCTAGATTAAACCATTTTTTCATTTTCC 3',
le fragment d'ADN obtenu a été digéré par PstI et XbaI et ligaturé dans le vecteur pVR1012 (exemple 1) linéarisé par PstI et XbaI, générant le plasmide pAB127 (7818 pb). Le gène gB code pour une protéine de 980 acides aminés.

### 6.1.2, pSB028: gène gB (forme ΔTM) cloné dans le vecteur pVR1012

Selon le profil d'hydropathie, le domaine trans-membranaire de la protéine gB de EHV-1 est positionné entre les résidus 801 et 875. Le plasmide contenant la forme tronquée du gène codant pour gB a été obtenu par la ligature des deux fragments d'ADN suivants : (a) le plasmide pAB127 (exemple 6.1.1.) digéré par AfeI et XbaI, et (b) un fragment de 276 pb obtenu par PCR à l'aide des oligonucléotides suivants :
SB125 (24 mer) (SEQ ID NO 51)
   5' AACAACAGAGGGTCGATAGAAGGC 3' et
SB126 (39 mer) (SED ID NO 52)
   5' AATTTTTCTAGATTACACGTTGACCACGCTGTCGATGTC 3'
en utilisant le plasmide pAB127 comme matrice et digéré par AfeI et XbaI. Le plasmide résultant, pSB028 (7279 pb) contient un gène gB de EHV-1 tronqué codant pour une protéine de 800 résidus.

### 6.2. Plasmides codant pour les différentes formes de EHV-1 gC

### 6.2.1. pAB129: gène gC (forme native) cloné dans le vecteur pVR1012

Le fragment d'ADN contenant la phase ouverte de lecture du gène gC de EHV-1 a été obtenu par PCR à l'aide des oligonucléotides suivants :
NS005 (31 mer) (SEQ ID NO 41)
   5' TTGTCGACATGTGGTTGCCTAATCTCGTGAG 3' et
NS006 (33 mer) (SEQ ID NO 42)
   5' TTGGATCCCTAAAAGTCAGACTTCTTGTACGGC 3'.
Ce produit PCR a été digéré par SaII et BamHI générant un fragment de 1412 pb, qui a ensuite été ligaturé dans le vecteur pVR1012 (exemple 1) linéarisé par la même double digestion. Le plasmide résultant, pAB129 (6281 pb) contient le gène codant pour la glycoprotéine gC de EHV-1 et ayant une taille de 468 résidus.

### 6.2.2. pSB029: gène gC (forme ΔTM) cloné dans le vecteur pVR1012

Selon le profil d'hydropathie, le domaine trans-membranaire de la protéine gC de EHV-1 est compris entre les résidus 429 et 455. Le plasmide contenant la forme tronquée du gène codant pour gC a été obtenu par la ligature des deux fragments d'ADN suivants : (a) le plasmide pAB129 (exemple 6.2.1.) linéarisé par une double digestion Aspl-BamHI et (b) un fragment de 287 pb obtenu par PCR à l'aide des oligonucléotides suivants :
SB127 (24 mer) (SEQ ID NO 53)
   5' GATCCGGAGGAGGAATACACACCC 3' et
SB128 (39 mer) (SEQ ID NO 54)
   5' AATTTTGGATCCCTAAACCGGCCTGTCCTCAACAATCGG 3'
en utilisant le plasmide pAB129 comme matrice et digéré par AspI et BamHI. Le plasmide résultant, pSB029 (6161 pb), contient un gène gC tronqué codant pour une protéine de 428 résidus.

### 6.3. Plasmides codant pour la forme tronquée de EHV-1 gD

### 6.3.1. pAB131: gène gD (forme native) cloné dans le vecteur pVR1012

Le fragment d'ADN contenant la phase ouverte de lecture du gène gD de EHV-1 a été obtenu par PCR à l'aide des oligonucléotides suivants :
NS007 (33 mer) (SEQ ID NO 43)
   5' TTGTCGACATGTCTACCTTCAAGCTTATGATGG 3' et
NS008 (32 mer) (SEQ ID NO 44)
   5' TTGGATCCTTACGGAAGCTGGGTATATTTAAC 3'.
Ce produit PCR a été digéré par SaII et BamHI générant un fragment de 1214 pb, qui a ensuite été ligaturé dans le vecteur pVR1012 (exemple 1) linéarisé par la même double digestion. Le plasmide résultant, pAB138 (6083 pb) contient le gène codant pour la glycoprotéine gD de 402 résidus.

### 6.3.2. pSB030: gène gD (forme ΔTM) cloné dans le vecteur pVR1012

Selon le profil d'hydropathie, le domaine trans-membranaire de la protéine gD de EHV-1 est compris entre les résidus 348 et 371. Le plasmide contenant la forme tronquée du gène codant pour gD a été obtenu par la ligature des trois fragments d'ADN suivants : (a) le plasmide pVR1012 (exemple 1) linéarisé par une double digestion SaII-BamHI, (b) le fragment de 825 pb issu de la digestion de pAB131 (exemple 6.3.1.) par SaII et BsmI et (c) un fragment de 239 pb obtenu par PCR à l'aide des oligonucléotides suivants :
SB129 (24 mer) (SEQ ID NO 55)
   5' CGGTTTCTTGGTGAATTCAACTTC 3' et
SB130 (42 mer) (SEQ ID NO 56)
   5' AATTTTGGATCCTTACGTAGAGTTGCTCTTAGACGTTTTTGG 3'
en utilisant le plasmide pAB131 comme matrice et digéré par BsmI et BamHI. Le plasmide résultant, pSB030 (5921 pb), contient un gène IgD tronqué codant pour une protéine de 347 résidus.

### Exemple 7: Plasmides codant pour les différentes formes des glycoprotéines de EHV-4

Les gènes codant pour les glycoprotéines gB, gC et gD de la souche KYT445/2 de EHV-4 ont été isolés par PCR à partir de l'ADN viral purifié.

### 7.1. Plasmides codant pour les différentes formes de EHV-4 gB

### 7.1.1. pAB136: gène gB (forme native) cloné dans le vecteur pVR1012

La phase codante du gène gB de EHV-4 a été amplifiée par PCR à l'aide des oligonucléotides suivants :
AB325 (35 mer) (SEQ ID NO 33)
   5' TTTCTGCAGATGTCCACTTGTTGCCGTGCTATTTG 3' et
AB326 (31 mer) (SEQ ID NO 34)
   5' TTTTCTAGATTAAACCATTTTTTCGCTTTCC 3',
le fragment d'ADN obtenu a été digéré par PstI et XbaI et ligaturé dans le vecteur pVR1012 (exemple 1) linéarisé par PstI et XbaI, générant le plasmide pAB136 (7801 pb). Le gène gB de EHV-4 code pour une protéine de 975 acides aminés.

### 7.1.2. pSB025: gène gB (forme ΔTM) cloné dans le vecteur pVR1012

Selon le profil d'hydropathie, le domaine trans-membranaire de la protéine gB de EHV-4 est positionné entre les résidus 797 et 867. Le plasmide contenant la forme tronquée du gène codant pour gB a été obtenu par la ligature des deux fragments d'ADN suivants : (a) le plasmide pAB136 (exemple 7.1.1.) digéré par SplI et Xbal, et (b) un fragment de 231 pb obtenu par PCR à l'aide des oligonuciéotides suivants :
SB119 (36 mer) (SEQ ID NO 45)
   5' TTTTGGTCTAGATTAGTCCACGTTGACAACGCTGTC 3' et
SB120 (23 mer) (SEQ ID NO 46)
   5' CGCAAGCTTATCGAGCCGTGCGC 3'
en utilisant le plasmide pAB136 comme matrice et digéré par SpII et XbaI. Le plasmide résultant, pSB025 (7264 pb) contient un gène gB tronqué codant pour une protéine de 796 résidus.

### 7.2. Plasmides codant pour les différentes formes de EHV-4 gC

### 7.2.1. pAB137: gène gC (forme native) cloné dans le vecteur pVR1012

Le fragment d'ADN contenant la phase ouverte de lecture du gène gC de EHV-4 a été obtenu par PCR à l'aide des oligonucléotides suivants :
AB327 (32 mer) (SEQ ID NO 35)
   5' TTTGTCGACATGGGTTTGGTAAATATAATGCG 3' et
AB328 (33 mer) (SEQ ID NO 36)
   5' TTTGGATCCTTAGAAGTCTGCTTTCTTGTAGGG 3'.
Ce produit PCR a été digéré par SaII et BamHI générant un fragment de 1463 pb, qui a ensuite été ligaturé dans le vecteur pVR1012 (exemple 1) linéarisé par la même double digestion. Le plasmide résultant, pAB137 (6330 pb) contient le gène codant pour la glycoprotéine gC de 485 résidus.

### 7.2.2. pSB026: gène gC (forme ΔTM) cloné dans le vecteur pVR1012

Selon le profil d'hydropathie, le domaine trans-membranaire de la protéine gC de EHV-4 est compris entre les résidus 425 et 472. Le plasmide contenant la forme tronquée du gène codant pour gC a été obtenu par la ligature des deux fragments d'ADN suivants : (a) le plasmide pAB137 (exemple 7.2.1.) linéarisé par une double digestion AclI-BamHI et (b) un fragment de 237 pb obtenu par PCR à l'aide des oligonucléotides suivants :
SB121 (24 mer) (SEQ ID NO 47)
   5' GTATCAATCCCAGCTGACCCCGAC 3' et
SB122 (41 mer) (SEQ ID NO 48)
   5' AATTTTGGATCCTTAGCCGTCCGGGTAACCCTCTATGATGC 3'
en utilisant le plasmide pAB137 comme matrice et digéré par AcII et BamHI. Le plasmide résultant, pSB026 (6147 pb), contient un gène gC tronqué codant pour une protéine de 424 résidus.

### 7.3. Plasmides codant pour la forme tronquée de EHV-4 gD

### 7.3.1. pAB138: gène gD (forme native) cloné dans le vecteur pVR1012

Le fragment d'ADN contenant la phase ouverte de lecture du gène gD de EHV-4 a été obtenu par PCR à l'aide des oligonucléotides suivants :
AB329 (33 mer) (SEQ ID NO 37)
   5' TTTGTCGACATGTCTACCTTCAAGCCTATGATG 3' et
AB330 (33 mer) (SEQ ID NO 38)
   5' TTTGGATCCTTACGGAAGCTGAGTATATTTGAC 3'.
Ce produit PCR a été digéré par SaII et BamHI générant un fragment de 1214 pb, qui a ensuite été ligaturé dans le vecteur pVR1012 (exemple 1) linéarisé par la même double digestion. Le plasmide résultant, pAB138 (6081 pb) contient le gène codant pour la glycoprotéine gD de 402 résidus.

### 7.3.2. pSB027: gène gD (forme ΔTM) cloné dans le vecteur pVR1012

Selon le profil d'hydropathie, le domaine trans-membranaire de la protéine gD de EHV-4 est compris entre les résidus 348 et 371. Le plasmide contenant la forme tronquée du gène codant pour gD a été obtenu par la ligature des deux fragments d'ADN suivants: (a) le plasmide pAB138 (exemple 7.3.1.) linéarisé par une double digestion EcoRI-BamHI et (b) un fragment de 310 pb obtenu par PCR à l'aide des oligonucléotides suivants :
SB123 (24 mer) (SEQ ID NO 49)
   5' TTTTCCGTAACAATTCCGAGCAGC 3' et
SB124 (39 mer) (SEQ ID NO 50)
   5' AATTTTGGATCCTTACGTAGAGTTGCTATTAGACGCTGG 3'
en utilisant le plasmide pAB138 comme matrice et digéré par EcoRI et BamHI. Le plasmide résultant, pSB027 (5919 pb), contient un gène gD tronqué codant pour une protéine de 347 résidus.

### Exemple 8 : Plasmide codant pour le GM-CSF canin

### 8.1. Préparation de l'ARN total de lymphocytes de chien stimulés in vitro par des mitogènes

Du sang de chien a été récolté sur un tube contenant de l'EDTA par une prise de sang réalisée sur une chienne Beagle. Les cellules mononucléées ont été récoltées par centrifugation sur un gradient de Ficoll, puis mises en culture en boîte de Petri de 60 mm de diamètre. Les cellules mononuclées de chien ont alors été stimulés avec de la concanavaline A (conA) (concentration finale d'environ 4 µg/ml) et avec de la phyto-hémagglutinine (PHA) (concentration finale d'environ 10 µg/ml). Après stimulation, les lymphoblastes " ConA " et " PHA " ont été récoltés par grattage des boîtes de culture, et l'ARN total de ces cellules a été extrait en utilisant le kit " mRNA isolation kit for White Blood Cells " (Boehringer Mannheim/Roche Cat # 1 934 325).

### 8.2. Isolement du gène codant pour le GM-CSF canin et construction du

### plasmide pJP074

L'ARN total extrait des lymphoblastes de chien stimulés par la ConA ou par la PHA (exemple 8.1.) a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'ACP avec les oligonucléotides suivants :
JP578 (SEQ ID NO 64) (33 mer)
   5' TATGCGGCCGCCACCATGTGGCTGCAGAACCTG 3'
et JP579 (SEQ ID NO 65) (36 mer)
   5' TATGCGGCCGCTACGTATCACTTCTTGACTGGTTTC 3'
pour amplifier un fragment ACP d'environ 450 paires de bases (pb). Ce fragment a été purifié par électrophorèse en gel d'agarose, puis ligaturé avec le vecteur pCR2.1 (InVitrogen, Carlsbad, CA, USA) pour donner le plasmide pJP074. La séquence du gène GM-CSF canin cloné dans le plasmide pJP074 a été trouvée équivalente à celle de la séquence du GM-CSF canin disponible sur GenBank (n° d'accès # S49738).

### 8.3. Construction du plasmide pJP084 et séquence du gène GM-CSF canin

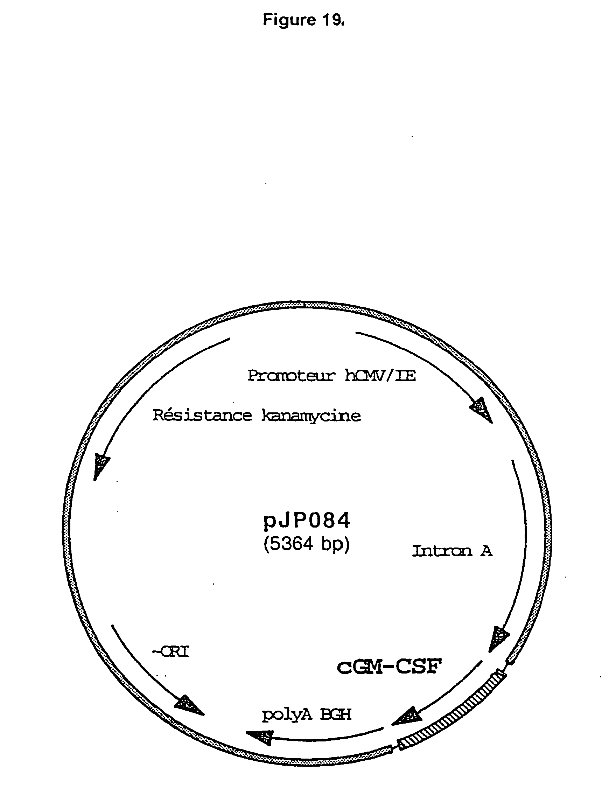

Le plasmide pJP074 (exemple 8.2.) a été digéré par NotI pour isoler, après électrophorèse en gel d'agarose, le fragment Notl-Notl d'environ 450 pb contenant le gène GM-CSF canin. Ce fragment a été ligaturé avec le plasmide pVR1012 (exemple 1). Le clone contenant la séquence GM-CSF canin (SEQ ID NO66, Figure N° 20) dans la bonne orientation par rapport au promoteur hCMV/IE a été identifié pJP084. Ce plasmide a une taille de 5364 pb (Figure N° 19).

### Exemple 9 : Plasmide codant pour le GM-CSF félin

### 9.1. Préparation de l'ARN total de lymphocytes de chat stimulés in vitro par des mitogènes

Du sang de chat a été récolté par prise de sang sur un tube contenant de l'EDTA. Les cellules mononucléées ont été récoltées par centrifugation sur un gradient de Ficoll, puis mises en culture en boîte de Petri de 60 mm de diamètre. Les cellules mononuclées de chat ont alors été stimulés avec de la concanavaline A (ConA) (concentration finale d'environ 4 µg/ml) et avec de la phyto-hémagglutinine (PHA) (concentration finale d'environ 10 µg/ml). Après stimulation, les lymphoblastes " ConA " et " PHA " ont été récoltés par grattage des boîtes de culture, et l'ARN total de ces cellules a été extrait en utilisant le kit " mRNA isolation kit for White Blood Cells " (Boehringer Mannheim/Roche Cat # 1 934 325).

### 9.2. Isolement du gène codant pour le GM-CSF félin et construction des plasmides pJ089 et pJP090

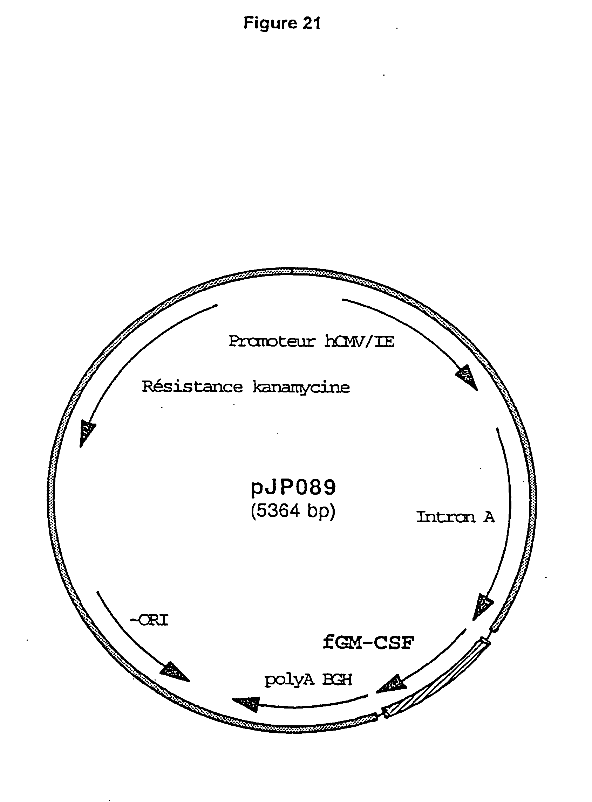

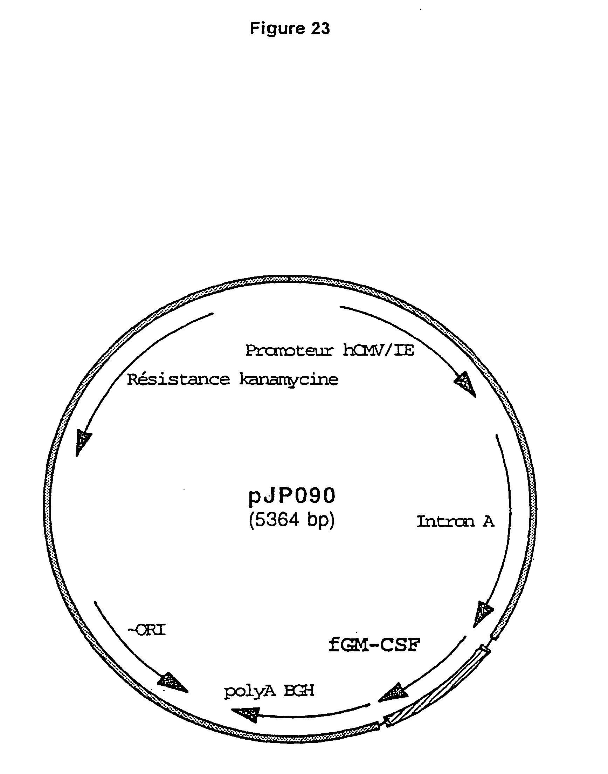

L'ARN total extrait des lymphoblastes de chat stimulés par la ConA et la PHA (exemple 9.1.) a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'ACP avec les oligonucléotides suivants :
JP578 (SEQ ID NO 64) (33 mer)
   5' TATGCGGCCGCCACCATGTGGCTGCAGAACCTG 3'
et JP579 (SEQ ID NO 65) (36 mer)
   5' TATGCGGCCGCTACGTATCACTTCTTGACTGGTTTC 3'
pour amplifier un fragment ACP d'environ 450 paires de bases (pb). Ce fragment a été digéré par NotI pour isoler, après électrophorèse en gel d'agarose, le fragment Notl-Notl de 450 pb. Ce fragment a été alors ligaturé avec le plasmide pVR1012 (exemple 1). Deux clones contenant la séquence GM-CSF félin (SEQ ID NO 67 et SEQ ID NO 68), dans la bonne orientation par rapport au promoteur hCMV/IE ont été identifiés respectivement pJP089 et pJP090. Ces deux plasmide ont une taille de 5364 pb (Figures N° 21 et 23).

La séquence du gène GM-CSF félin cloné dans le plasmide pJP089 contient 13 différences au niveau nucléotidique avec la séquence GM-CSF félin disponible sur GenBank (n° d'accès AF053007). Le changement le plus important est une changement C → T qui entraîne un changement Leucine → Phenylalanine pour l'acide aminé (première base du codon de l'acide aminé # 107 ; Figure N° 22). La séquence du gène GM-CSF félin cloné dans le plasmide pJP090 est équivalent à celle contenue dans le plasmide pJP089, sauf que le changement Leucine → Phenylalanine au n'existe pas pour l'acide aminé # 107 (Figure N° 24). La vérification de la séquence 3' du gène GM-CSF félin au moyen du kit 3' RACE a montré que, à cette position 107, on peut avoir chez le même chat, l'acide aminé Leucine ou l'acide aminé Phenylalanine.

### Exemple 10 : Plasmide codant pour le GM-CSF équin

### 10.1. Préparation de l'ARN total de lymphocytes de cheval stimulés in vitro par des mitogènes

Du sang de cheval a été récolté sur un tube contenant de l'EDTA par une prise de sang à la veine jugulaire. Les cellules mononucléées ont été récoltées par centrifugation sur un gradient de Ficoll, puis mises en culture en boîte de Petri de 60 mm de diamètre. Les cellules mononuclées de cheval ont alors été stimulées soit avec de la concanavaline A (ConA) (concentration finale d'environ 5 µg/ml) soit avec de la phyto-hémagglutinine (PHA) (concentration finale d'environ 10 µg/ml). Après stimulation, les lymphoblastes " ConA " et " PHA " ont été récoltées par grattage des boîtes de culture, et l'ARN total de ces cellules a été extrait en utilisant le kit " mRNA isolation kit for White Blood Cells " (Boehringer Mannheim/Roche Cat # 1 934 325).

### 10.2. Isolement du gène codant pour le GM-CSF équin

L'ARN total extrait des lymphoblastes de cheval stimulés par la ConA ou par la PHA (exemple 10.1.) a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'ACP avec les oligonucléotides suivants :
JP734 (SEQ ID NO 70) (44 mer)
   5' CATCATCATGTCGACGCCACCATGTGGCTGCAGAACCTGCTTCT 3'
et JP735 (SEQ ID NO 71) (41 mer)
   5' CATCATCATGCGGCCGCTACTTCTGGGCTGCTGGCTTCCAG 3'
pour amplifier un fragment ACP d'environ 500 paires de bases (pb). Ce fragment a été purifié par électrophorèse en gel d'agarose.

### 10.3. Construction du plasmide pJP097 et séquence du gène GM-CSF équin

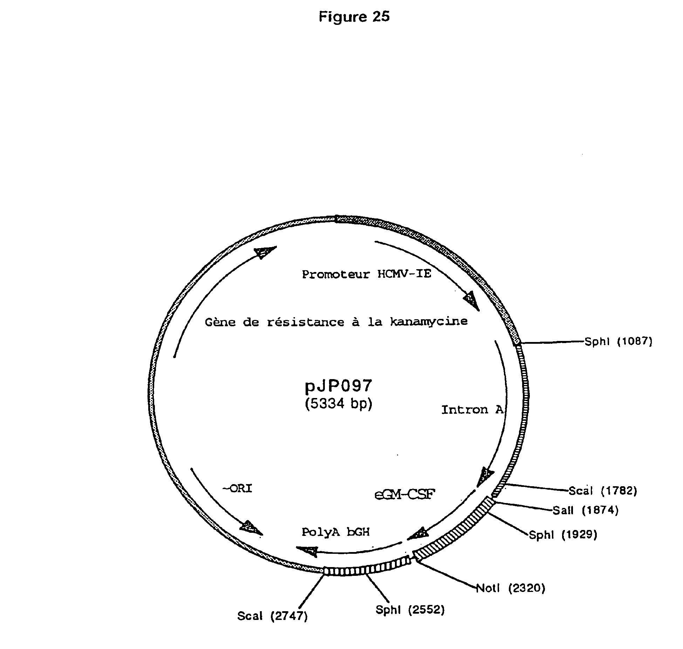

Le fragment ACP purifié obtenu à l'exemple 10.2. a été digéré par Notl pour isoler, après électrophorèse en gel d'agarose, le fragment Notl-Notl d'environ 450 pb contenant le gène GM-CSF équin. Ce fragment a été ligaturé avec le plasmide pVR1012 (exemple 1). Le clone contenant la séquence GM-CSF équin (SEQ ID NO 69, Figure N° 26) dans la bonne orientation par rapport au promoteur hCMV/IE a été identifié pJP097. Ce plasmide a une taille de 5334 pb (Figure N° 25).

### Exemple 11 : Tableau récapitulatif des plasmides

| Pathogène | Antigène | Nb. de résidus forme native | Domaine TM | Nb de résidus forme tronquée | Plasmides d'expression |
|---|---|---|---|---|---|
| CDV | F | 662 | 606-626 | 605 | pPB229 et pNS021* |
| | HA | 607 | 1-60** | 574 | pNS018 et pNS024* |
| | | | | | |
| CPI-2 | F | 529 | 474-517 | 473 | pAB115 et pSB032* |
| | HN | 565 | 1-40** | 557 | pAB114 et pSB034* |
| | | | | | |
| CHV-1 | gB | 878 | 702-769 | 701 | pAB037 et pSB016* |
| | gC | 459 | 422-452 | 421 | pSB018 et pSB019* |
| | gD | 345 | 310-328 | 309 | pAB038 et pSB017* |
| | | | | | |
| FHV-1 | gB | 949 | 761-834 | 760 | pSB020 et pSB021* |
| | gC | 534 | 495-526 | 494 | pSB022 et pSB023* |
| | gD | 374 | 328-353 | 327 | pAB029 et pSB024* |
| | | | | | |
| EHV-1 | gB | 980 | 801-875 | 800 | pAB127 et pSB028* |
| | gC | 468 | 429-455 | 428 | pAB129 et pSB029* |
| | gD | 402 | 348-371 | 347 | pAB131 et pSB030* |
| | | | | | |
| EHV-4 | gB | 975 | 797-867 | 796 | pAB136 et pSB025* |
| | gC | 485 | 425-472 | 424 | pAB137 et pSB026* |
| | gD | 402 | 348-371 | 347 | pAB138 et pSB027* |

| | | | | | |
|---|---|---|---|---|---|
| * Plasmides codant pour les formes tronquées des antigènes ** Cas particulier des gènes HA de CDV et HN de CPI-2 : domaine trans-membranaire et séquence signal confondus | | | | | |

### Exemple 12 : Méthodes de biologie moléculaire

### Culture et purification des virus

Les virus ont été cultivés sur des systèmes cellulaires appropriés jusqu'à obtention d'un effet cythopathique. Les systèmes cellulaires à utiliser pour chaque virus sont bien connus de l'homme du métier. Brièvement, les cellules appropriées ont été infectées par la souche virale étudiée à une multiplicité d'infection de 1 et ont été incubées à 37°C pendant le temps nécessaire à l'obtention d'un effet cythopathique (en moyenne 36 heures).

Dans le cas des virus à ADN, après la culture, le surnageant et les cellules lysées ont été récoltés et les débris cellulaires ont été éliminés par une centrifugation à 1000 g et à 4°C pendant 10 minutes. Les particules virales ont été récoltées par ultracentrifugation à 400 000 g et 4°C pendant 1 heure. Les culots ont été repris dans un volume minimum de tampon (Tris 10 mM, EDTA 1 mM).

Les virus à ARN ont été purifiés selon les techniques standard de purification bien connues de l'homme du métier.

### Extraction d'ADN génomique viral

Les suspensions virales concentrées ont été traitées par la protéinase K (100 mg/ml final) en présence de sodium dodecyl sulfate (SDS) (0.5% final) pendant 2 heures à 37 °C. L'ADN viral a ensuite été extrait à l'aide d'un mélange phénol/chloroforme, puis précipité avec deux volumes d'éthanol absolu à -20°C pendant 16 heures et ensuite centrifugé à 10 000 g pendant 15 minutes à 4°C. Les culots d'ADN ont été séchés, puis repris dans un volume minimum d'eau ultrapure stérile.

### Isolement d'ARN génomique viral

L'ARN génomique de chaque virus a été extrait en utilisant la technique du " thiocyanate de guanidinium/phénol-chloroforme" décrite par P. Chomczynski et N. Sacchi (Anal. Biochem. 1987. 162. 156-159).

### Techniques de biologie moléculaire

Toutes les constructions de plasmides ont été réalisées en utilisant les techniques standard de biologie moléculaire décrites par Sambrook et al. (Molecular Cloning : A Laboratory Manual. 2nd Edition Cold Spring Harbor Laboratory, Cold Spring Harbor, New York, 1989). Tous les fragments de restriction utilisés pour la présente invention ont été isolés à l'aide du kit " Geneclean " (BIO101 Inc., La Jolla, CA). Pour toutes les constructions, les fragments d'ADN clonés, ainsi que les jonctions avec le vecteur d'expression, ont été séquencés par la méthode de Sanger (Sambrook et al., 1989).

### PCR et RT-PCR

Les oligonucléotides spécifiques des gènes ou des fragments de gènes clonés ont été synthétisés, certains d'entre eux comportant dans certains cas à leur extrémité 5' des sites de restriction facilitant le clonage des fragments amplifiés. Les réactions de transription inverse (RT) et l'amplification en chaîne par polymérase (PCR) ont été effectuées selon des techniques standard (Sambrook et al., 1989).

### Purification de plasmides à grande échelle

La production, à l'échelle de la dizaine de mg, de plasmides purifiés entrant dans les compositions vaccinales a été effectuée par la méthode des gradients de chlorure de césium-bromure d'éthidium (Sambrook et al, 1989).

### Exemple 13 : Formulation des plasmides vaccinaux

La solution d'ADN contenant un ou plusieurs plasmides selon les exemples 2 à 10 est concentrée par précipitation éthanolique comme décrit dans Sambrook et al (1989). Le culot d'ADN est repris par une solution de NaCl 0.9% de façon à obtenir une concentration de 1 mg/ml. Une solution de DMRIE-DOPE à 0,75mM est préparée par reprise d'un lyophilisat de DMRIE-DOPE par un volume adapté d'H₂O stérile.

La formation des complexes ADN plasmidique-lipide est réalisée par dilution à parties égales de la solution de DMRIE-DOPE 0.75 mM par la solution d'ADN à 1 mg/ml dans NaCl 0.9%. La solution d'ADN est introduire progressivement à l'aide d'une aiguille sertie 26G le long de la paroi du flacon contenant la solution de lipide cationique de façon à éviter la formation de mousse. On procède à une agitation douce dès que les deux solutions ont été mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes avant de procéder à l'immunisation des animaux.

### Exemple 14 : Immunisation des chiens contre CDV

Une injection de 2 ml par voie sous-cutanée ou intramusculaire répétée à 28 jours plus tard. La masse totale de plasmide utilisée lors de chaque immunisation est de 100, 500, 1000 ou 2000 µg selon les vaccins. L'homme de l'art possède les connaissances nécessaires pour adapter le volume ou la concentration en fonction de la dose de plasmide requise.

### 14.1. Epreuve virulente

La souche d'épreuve utilisée correspond à un broyat de rate prélevée sur un chien infecté par le virus de la maladie de Carré, souche Snyder Hill et diluée au centième en tampon PBS. La dilution est conservée sur de la glace pilée jusqu'à utilisation.

Après anesthésie générale, on administre la souche d'épreuve diluée au centième par voie intracraniale sous un volume de 0,5 ml, 49 jours après la première injection.

### 14.2. Suivi clinique post-épreuve

Ce suivi clinique quotidien a été réalisé pendant les 21 jours suivant l'épreuve et a compris pour chaque chien un examen clinique pour détecter d'éventuels signes cliniques de la maladie de Carré et une prise de la température rectale.

L'examen clinique comprend :
- Une observation de l'état général de l'animal sur une échelle à 4 niveaux :
   " bon " avec un score de 0, " apathie " avec un score de 1, " dépression " avec un score de 2 et " prostration " avec un score de 3. La mort de l'animal équivaut à un score clinique de 10.
- Une évaluation des symptômes oculo-nasaux (recherche d'écoulement séreux ou purulent, de rhinite et/ou conjonctivite). Une conjonctivite/rhinite avec un écoulement nasal séreux équivaut à un score de 1, celle avec un écoulement nasal purulent équivaut à un score de 2.
- Une évaluation des symptômes digestifs (recherche de signes de gastroentérite). Une gastroentérite légère équivaut à un score de 1, une sévère à un score de 2.
- Une évaluation des symptômes nerveux (recherche de myoclonies, convulsions et/ou paralysie). Une myoclonie équivaut à un score de 1, une convulsion à un score de 2 et une paralysie à un score de 3.
- Le suivi de la température corporelle de l'animal.

Une température inférieure ou égale à 37,5°C équivaut à un score de 3, une température comprise entre 37,5°C et 39,5°C équivaut à un score de 0, une température égale à 39,5°C ou comprise entre 39,5°C et 40°C équivaut à un score de 1, une température égale à 40°C ou comprise entre 40°C et 40,5°C équivaut à un score de 2, et enfin une température supérieure à 40,5°C équivaut à un score de 3.

Sur les animaux ayant succombé à l'épreuve, un prélèvement de rate est réalisé pour la mise en évidence du virus de Carré par immunofluorescence.

Un score clinique global a été calculé de la manière suivante : pour chaque signe clinique a été établi un score moyen par groupe d'animaux sur la période d'observation et le score totale est la somme des scores moyens pour les cinq signes cliniques considérés.

Résultats des expériences d'épreuves après immunisation des chiens par la voie intramusculaire :

| Plasmide | Formulation | Antigènes | Dose | Cytokine | Mortalité | Score clinique | Réponse en anticorps* |
|---|---|---|---|---|---|---|---|
| Contrôle | --- | --- | --- | --- | 4/4 | 26,1 | 0,3 +/- 0,3 |
| | | | | | | | |
| pNS018 | --- | HA natif | 50 µg | --- | 4/5 | 21,6 | 0,3 +/- 0,2 |
| pPB229 | | F natif | 50 µg | | | | |
| pNS018 | DMRIE- | HA natif | 50 µg | --- | 1/5 | 7,8 | 0,8 +/- 0,3 |
| pPB229 | DOPE | F natif | 50 µg | | | | |
| pNS024 | DMRIE- | HA optimisé | 50 µg | --- | 1/5 | 7,2 | 1,9 +/- 0,8 |
| pNS021 | DOPE | F optimisé | 50 µg | | | | |
| pNS024 | DMRIE- | HA optimisé | 500µg | --- | 0/5 | 0,6 | 1,5 +/- 0,3 |
| pNS021 | DOPE | F optimisé | 500 µg | | | | |
| Contrôle | --- | --- | --- | --- | 4/4 | 25,0 | 0,2 +/- 0,0 |
| | | | | | | | |
| pNS018 | DMRIE-DOPE | HA natif | 50 µg | --- | 5/5 | 24,4 | 0,2 +/- 0,0 |
| pPB229 | | F natif | 50 µg | | | | |
| pNS018 | DMRIE-DOPE | HA natif | 500 µg | --- | 3/5 | 19.0 | 0,4 +/- 0,2 |
| pPB229 | | F natif | 500 µg | | | | |
| pNS024 | DMRIE-DOPE | HA optimisé | 500 µg | --- | 1/5 | 9,2 | 0,6 +/- 0,7 |
| pNS021 | | F optimisé | 500 µg | | | | |
| pNS024 | DMRIE-DOPE | HA optimisé | 500 µg | | 0/5 | 2,4 | 1,7 +/- 0,9 |
| pNS021 | | F optimisé | 500 µg | | | | |
| pJP084 | | | 200 µg | GM-CSF | | | |
| pNS024 | DMRIE-DOPE | HA optimisé | 500 µg | --- | 0/5 | 0,8 | 1,4 +/-1,0 |
| pNS024 | DMRIE-DOPE | HA optimisé | 500 µg | --- | 0/5 | 5 | 1,4+/-1,3 |
| pNS021 | | F optimisé | 500 µg | | | | |
| pNS016 | | M natif | 500 µg | | | | |
| pNS017 | | N natif | 500 µg | | | | |

| | | | | | | | |
|---|---|---|---|---|---|---|---|
| * titre moyen +/- écart-type | | | | | | | |

Les plasmides pNS016 et pNS017 insérant respectivement le gène natif M et le gène natif N, ont été construits de la même façon que le plasmide pNS018.

Il est surprenant de constater que le résultat de protection obtenu avec HA optimisé seul est supérieur ou égal aux résultats obtenus avec HA et F optimisés ou avec HA et F optimisés + M et N natifs.

## Revendications

1. Vaccin ADN contre le virus de la maladie de Carré (CDV) affectant les chiens, comprenant un plasmide contenant une séquence nucléotidique codant pour un immunogène de CDV, et les éléments nécessaires a son expression *in vivo,* un lipide cationique, (N-(2-hydroxyéthyl)-N,N-diméthyl-2,3-bis(tétradecyloxy)-1 propaneammonium (DMRIE) et du dioléoylphosphatidyléthanolamine (DOPE), dans lequel le vaccin ADN administré par voie intramusculaire ou sous-cutanée induit une réponse immunitaire efficace et protectrice chez le chien contre CDV.

2. Vaccin selon la revendication 1, comprenant en outre une protéine GM-CSF canine.

3. Vaccin selon l'une quelconque des revendications 1 à 2, comprenant en outre un vecteur d'expression contenant une séquence nucléotidique codant pour la protéine GM-CSF canine dans des conditions permettant l'expression in vivo de cette séquence.

4. Vaccin selon la revendication 3, dans lequel le vecteur d'expression est un plasmide.

5. Vaccin selon l'une quelconque des revendications 1 à 4, dans lequel la séquence nucléotidique codant pour un immunogène de CDV est la séquence d'un gène dont on a délété la partie codant pour le domaine transmembranaire.

6. Vaccin selon l'une quelconque des revendications 1 à 5, dans lequel le plasmide comportant la séquence nucléotidique codant pour un immunogène de CDV comporte aussi une séquence nucléotidique codant pour un signal tPA.

7. Vaccin selon l'une quelconque des revendications 1 à 6, dans lequel le plasmide comportant la séquence nucléotidique codant pour un immunogène de CDV comporte aussi un intron stabilisateur.

8. Vaccin selon la revendication 7, dans lequel l'intron stabilisateur est l'intron II du gène de la bêta-globine du lapin.

9. Vaccin selon l'une quelconque des revendications 1 à 8, dans lequel le plasmide contient une séquence nucléotidique codant pour l'antigène HA de CDV.

10. Vaccin selon la revendication 9, dans lequel la séquence nucléotidique codant pour l'antigène HA est optimisée par la substitution de la séquence codant pour le signal de HA par une séquence nucléotidique codant pour un signal tPA, par la délétion du fragment de la séquence nucléotidique codant pour le domaine transmembranaire de HA, par l'insertion d'un intron en amont de la séquence nucléotidique codant pour HA ou par une combinaison de ces modifications.

11. Vaccin selon l'une quelconque des revendications 1 à 8, dans lequel le plasmide contient une séquence nucléotidique codant pour l'antigène F de CDV.

12. Vaccin selon la revendication 11, dans lequel la séquence nucléotidique codant pour l'antigène F est optimisée par la substitution de la séquence codant pour le signal de F par une séquence nucléotidique codant pour un signal tPA, par la délétion du fragment de la séquence nucléotidique codant pour le domaine transmembranaire de F, par l'insertion d'un intron en amont de la séquence nucléotidique codant pour F ou par une combinaison de ces modifications.

13. Vaccin selon la revendication 10 ou la revendication 12, dans lequel le signal tPA est le signal tPA humain.

14. Vaccin selon la revendication 10 ou la revendication 12, dans lequel l'intron est l'intron II de la bêta globine du lapin.

15. Vaccin selon l'une quelconque des revendications 9 à 14, comprenant en outre, dans le même plasmide ou dans un autre plasmide, une séquence nucléotidique codant pour la protéine M ou la protéine N de CDV.

16. Vaccin selon l'une quelconque des revendications 9 à 15, comprenant un premier plasmide d'expression contenant une séquence nucléotidique codant pour l'antigène HA de CDV optimisée par la substitution de la séquence codant pour le signal de HA par une séquence nucléotidique codant pour le signal tPA humain, par la délétion du fragment de la séquence nucléotidique codant pour le domaine transmembranaire de HA et par l'insertion de l'intron II du gène de la bêta-globine du lapin en amont de la séquence nucléotidique codant pour HA, et d'un deuxième plasmide d'expression contenant une séquence nucléotidique codant pour l'antigène F de CDV optimisée par la délétion du fragment de la séquence nucléotidique codant pour le domaine transmembranaire de F et par l'insertion de l'intron II de la bêta-globine du lapin en amont de la séquence nucléotidique codant pour F.

17. Vaccin selon la revendication 16, comprenant en outre un plasmide d'expression codant pour le GM-CSF canin.

## Claims

1. A DNA vaccine against the canine distemper virus (CDV) affecting dogs, comprising a plasmid containing a nucleotide sequence coding for a CDV immunogen, and the elements necessary for its expression *in vivo,* a cationic lipid, (N-(2-hydroxyethyl)-N,N-dimethyl-2,3-bis(tetradecyloxy)-1 propane ammonium (DMRIE) and dioleolyphosphatidylethanol amine (DOPE), in which the DNA vaccine administered intramuscularly or subcutaneously induces an effective immunitary response protective in a dog against CDV.

2. A vaccine according to claim 1 further comprising a canine GM-CSF protein.

3. A vaccine according to either one of claims 1 and 2 and further comprising an expression vector containing a nucleotide sequence coding for the canine GM-CSF protein under conditions permitting *in vivo* expression of said sequence.

4. A vaccine according to claim 3 wherein the expression vector is a plasmid.

5. A vaccine according to any one of claims 1 to 4 wherein the nucleotide sequence coding for a CDV immunogen is the sequence of a gene from which the part coding for the transmembrane domain has been deleted.

6. A vaccine according to any one of claims 1 to 5 wherein the plasmid comprising the nucleotide sequence coding for a CDV immunogen also comprises a nucleotide sequence coding for a tPA signal.

7. A vaccine according to any one of claims 1 to 6 wherein the plasmid comprising the nucleotide sequence coding for a CDV immunogen also comprises a stabilising intron.

8. A vaccine according to claim 7 wherein the stabilising intron is intron II of the gene of rabbit beta-globin.

9. A vaccine according to any one of claims 1 to 8 wherein the plasmid contains a nucleotide sequence coding for the HA antigen of CDV.

10. A vaccine according to claim 9 wherein the nucleotide sequence coding for the HA antigen is optimised by the substitution of the sequence coding for the HA signal by a nucleotide sequence coding for a tPA signal, by the deletion of the fragment of the nucleotide sequence coding for the HA transmembrane domain, by the insertion of an intron upstream of the nucleotide sequence coding for HA or by a combination of such modifications.

11. A vaccine according to any one of claims 1 to 8 wherein the plasmid contains a nucleotide sequence coding for the F antigen of CDV.

12. A vaccine according to claim 11 wherein the nucleotide sequence coding for the F antigen is optimised by the substitution of the sequence coding for the F signal by a nucleotide sequence coding for a tPA signal, by the deletion of the fragment of the nucleotide sequence coding for the F transmembrane domain, by the insertion of an intron upstream of the nucleotide sequence coding for F or by a combination of such modifications.

13. A vaccine according to claim 10 or claim 12 wherein the tPA signal is the human tPA signal.

14. A vaccine according to claim 10 or claim 12 wherein the intron is intron II of rabbit beta-globin.

15. A vaccine according to any one of claims 9 to 14 further comprising in the same plasmid or in another plasmid a nucleotide sequence coding for the protein M or the protein N of CDV.

16. A vaccine according to any one of claims 9 to 15 comprising a first expression plasmid containing a nucleotide sequence coding for the HA antigen of CDV optimised by the substitution of the sequence coding for the HA signal by a nucleotide sequence coding for the human tPA signal, by the deletion of the fragment of the nucleotide sequence coding for the HA transmembrane domain and by the insertion of intron II of the gene of rabbit beta-globin upstream of the nucleotide sequence coding for HA, and a second expression plasmid containing a nucleotide sequence coding for the F antigen of CDV optimised by the deletion of the fragment of the nucleotide sequence coding for the F transmembrane domain and by the insertion of intron II of rabbit beta-globin upstream of the nucleotide sequence coding for F.

17. A vaccine according to claim 16 and further comprising an expression plasmid coding for canine GM-CSF.

## Patentansprüche

1. DNA-Impfstoff gegen das Staupevirus, das Hunde befällt, (CDV) umfassend ein Plasmid, das eine ein CDV-Immunogen codierende Nucleotidsequenz enthält, und die Elemente, die zu seiner *in vivo*-Expression erforderlich sind, ein kationisches Lipid, (N-(2-hydroxyethyl)-N,N-dimethyl-2,3-bis(tetradecyloxy)-1-Propanammonium (DMRIE) und Dioleoylphosphatidylethanolamin (DOPE), wobei der intramuskulär oder subkutan verabreichte DNA-Impfstoff beim Hund eine wirksame und schützende Immunantwort gegen CDV induziert.

2. Impfstoff nach Anspruch 1, ferner umfassend ein canines GM-CSF-Protein.

3. Impfstoff nach einem der Ansprüche 1 bis 2, ferner umfassend einen Expressionsvektor, der eine das canine GM-CSF-Protein codierende Nucleotidsequenz enthält, unter Bedingungen, die die *in vivo*-Expression dieser Sequenz erlauben.

4. Impfstoff nach Anspruch 3, wobei der Expressionsvektor ein Plasmid ist.

5. Impfstoff nach einem der Ansprüche 1 bis 4, wobei die ein CDV-Immunogen codierende Nucleotidsequenz die Sequenz eines Gens ist, in dem der die transmembrane Region codierende Teil deletiert wurde.

6. Impfstoff nach einem der Ansprüche 1 bis 5, wobei das Plasmid, das die ein CDV-Immunogen codierende Nucleotidsequenz umfasst, auch eine Nucleotidsequenz umfasst, die ein tPA-Signal codiert.

7. Impfstoff nach einem der Ansprüche 1 bis 6, wobei das Plasmid, das die ein CDV-Immunogen codierende Nucleotidsequenz umfasst, auch ein stabilisierendes Intron umfasst.

8. Impfstoff nach Anspruch 7, wobei das stabilisierende Intron das Intron II des Kaninchen-beta-Globin-Gens ist.

9. Impfstoff nach einem der Ansprüche 1 bis 8, wobei das Plasmid eine Nucleotidsequenz enthält, die das HA-Antigen von CDV codiert.

10. Impfstoff nach Anspruch 9, wobei die das HA-Antigen codierende Nucleotidsequenz optimiert ist durch Substitution der das HA-Signal codierenden Sequenz durch eine Nucleotidsequenz, die ein tPA-Signal codiert, durch Deletion des Fragments der Nucleotidsequenz, das die transmembrane Domäne von HA codiert, durch Insertion eines Introns stromaufwärts der HA codierenden Nucleotidsequenz oder durch eine Kombination dieser Modifizierungen.

11. Impfstoff nach einem der Ansprüche 1 bis 8, wobei das Plasmid eine Nucleotidsequenz enthält, die das F-Antigen von CDV codiert.

12. Impfstoff nach Anspruch 11, wobei die das F-Antigen codierende Nucleotidsequenz optimiert ist durch Substitution der das F-Signal codierenden Sequenz durch eine Nucleotidsequenz, die das tPA-Signal codiert, durch Deletion des Fragments der Nucleotidsequenz, das die transmembrane Domäne von F codiert, durch Insertion eines Introns stromaufwärts der F codierenden Nucleotidsequenz oder durch eine Kombination dieser Modifizierungen.

13. Impfstoff nach Anspruch 10 oder Anspruch 12, wobei das tPA-Signal das humane tPA-Signal ist.

14. Impfstoff nach Anspruch 10 oder Anspruch 12, wobei das Intron das Intron II des Kaninchen-beta-Globin-Gens ist.

15. Impfstoff nach einem der Ansprüche 9 bis 14, der ferner im selben Plasmid oder in einem anderen Plasmid eine Nucleotidsequenz umfasst, die das Protein M oder das Protein N von CDV codiert.

16. Impfstoff nach einem der Ansprüche 9 bis 15, umfassend ein erstes Expressionsplasmid, das eine das HA-Antigen von CDV codierende Nucleotidsequenz enthält, die optimiert ist durch Substitution der das HA-Signal codierenden Sequenz durch eine Nucleotidsequenz, die das humane tPA-Signal codiert, durch Deletion des Fragments der Nucleotidsequenz, das die transmembrane Domäne von HA codiert, und durch Insertion des Introns II des Kaninchen-beta-Globin-Gens stromaufwärts der HA codierenden Nucleotidsequenz, und ein zweites Expressionsplasmid, das eine das F-Antigen von CDV codierende Nucleotidsequenz enthält, die optimiert ist durch Deletion des Fragments der Nucleotidsequenz, das die transmembrane Domäne von F codiert, und durch Insertion des Introns II des Kaninchen-beta-Globin-Gens stromaufwärts der F codierenden Nucleotidsequenz.

17. Impfstoff nach Anspruch 16, ferner umfassend ein Expressionsplasmid, das das canine GM-CSF codiert.


### LISTE DE SEQUENCES

<110> MERIAL
<120> Vaccin ADN pets amélioré
<130> Vaccin ADN pets amélioré
<140> numéro demande
   <141> date demande
<160> 73
<170> PatentIn Ver. 2.1
<210> 1
   <211> 21
   <212> ADN
   <213> Artificial Sequence
<220>
   <223> Description of Artificial Sequence: oligonucléotide
<400> 1
   atgagcccac tcttacaaca a 21
<210> 2
   <211> 35
   <212> ADN
   <213> Artificial Sequence
<220>
   <223> Description of Artificial Sequence: oligonucléotide
<400> 2
   tttcgcggat ccattaaagg aagagcgcct aaccg 35
<210> 3
   <211> 30
   <212> ADN
   <213> Artificial Sequence
<220>
   <223> Description of Artificial Sequence: oligonucléotide
<400> 3
   tttcgcggat cccacaaagt atcaactagc 30
<210> 4
   <211> 23
   <212> ADN
   <213> Artificial Sequence
<220>
   <223> Description of Artificial Sequence: oligonucléotide
<400> 4
   gggatttgct gccgatgcaa tag 23
<210> 5
   <211> 33
   <212> ADN
   <213> Artificial Sequence
<220>
   <223> Description of Artificial Sequence: oligonucléotide
<400> 5
   tttacgcgtc gacttgggga cccttgattg ttc 33
<210> 6
   <211> 36
   <212> ADN
   <213> Artificial Sequence
<220>
   <223> Description of Artificial Sequence: oligonucléotide
<400> 6
   tttacgcgtc gacctgtagg aaaaagaaga aggcat 36
<210> 7
   <211> 20
   <212> ADN
   <213> Artificial Sequence
<220>
   <223> Description of Artificial Sequence: oligonucléotide
<400> 7
   ttggggaccc ttgattgttc 20
<210> 8
   <211> 21
   <212> ADN
   <213> Artificial Sequence
<220>
   <223> Description of Artificial Sequence: oligonucléotide
<400> 8
   ctgtaggaaa aagaagaagg c 21
<210> 9
   <211> 40
   <212> ADN
   <213> Artificial Sequence
<220>
   <223> Description of Artificial Sequence: oligonucléotide
<400> 9
   gatctgcagc acgtgtctag aggatatcga attcgcggcc 40
<210> 10
   <211> 40
   <212> ADN
   <213> Artificial Sequence
<220>
   <223> Description of Artificial Sequence: oligonucléotide
<400> 10
   gatccgcggc cgcgaattcg atatcctcta gacacgtgct 40
<210> 11
   <211> 30
   <212> ADN
   <213> Artificial Séquence
<220>
   <223> Description of Artificial Sequence: oligonucléotide
<400> 11
   ttctgcagat gctctcctac caagayaagg 30
<210> 12
   <211> 28
   <212> ADN
   <213> Artificial Sequence
   <220>
<223> Description of Artificial Sequence: oligonucléotide
<400> 12
   ttgtcgacat gtgtatcatc atmctgtc 28
<210> 13
   <211> 26
   <212> ADN
   <213> Artificial Sequence
<220>
   <223> Description of Artificial Sequence: oligonucléotide
<400> 13
   tttctagaca gccgagcccc atgcac 26
<210> 14
   <211> 30
   <212> ADN
   <213> Artificial Sequence
<220>
   <223> Description of Artificial Sequence: oligonucléotide
<400> 14
   ttggatccga tatatgacca gaatacttca 30
<210> 15
   <211> 22
   <212> ADN
   <213> Artificial Sequence
<220>
   <223> Description of Artificial Séquence: oligonucléotide
<400> 15
   tatattgaag gacaacttgg gg 22
<210> 16
   <211> 36
   <212> ADN
   <213> Artificial Sequence
<220>
   <223> Description of Artificial Sequence: oligonucléotide
<400> 16
   ctagtctaga ttaattatta tcaactttta caacac 36
<210> 17
   <211> 25
   <212> ADN
   <213> Artificial Séquence
<220>
   <223> Description of Artificial Sequence: oligonucléotide
<400> 17
   cgagaaactt gttatttttc taaag 25
<210> 18
   <211> 51
   <212> ADN
   <213> Artificial Sequence
<220>
   <223> Description of Artificial Sequence: oligonucléotide
<400> 18
   ataagaatgc ggccgcaaag gctatatatt ttttggggta ttatttattg g 51
<210> 19
   <211> 32
   <212> ADN
   <213> Artificial Sequence
<220>
   <223> Description of Artificial Sequence: oligonucléotide
<400> 19
   aaaactgcag atgagtttta aaaattttta tc 32
<210> 20
   <211> 30
   <212> ADN
   <213> Artificial Sequence
<220>
   <223> Description of Artificial Sequence: oligonucléotide
<400> 20
   ctagtctaga ttagatctta ttattttttg 30
<210> 21
   <211> 24 <212> ADN
   <213> Artificial Sequence
<220>
   <223> Description of Artificial Sequence: oligonucléotide
<400> 21
   tggattgacg gtcttataac acgc 24
<210> 22
   <211> 37
   <212> ADN
   <213> Artificial Sequence
<220>
   <223> Description of Artificial Sequence: oligonucléotide
<400> 22
   ctagtctaga ttaattttca tccgatgcat caaacac 37
<210> 23
   <211> 24
   <212> ADN
   <213> Artificial Sequence
<220>
   <223> Description of Artificial Sequence: oligonucléotide
<400> 23
   ctgtggacag agaccctaaa actc 24
<210> 24
   <211> 50
   <212> ADN
   <213> Artificial Sequence
<220>
   <223> Description of Artificial Sequence: oligonucléotide
<400> 24
   tttccttttg cggccgctta tatgctgtct atatcataaa attttaaggc 50
<210> 25
   <211> 24
   <212> ADN
   <213> Artificial Sequence
<220>
   <223> Description of Artificial Sequence: oligonucléotide
<400> 25
   gatcgtcccg ccataccgtc tggg 24
<210> 26
   <211> 39
   <212> ADN
   <213> Artificial Sequence
<220>
   <223> Description of Artificial Sequence: oligonucléotide
<400> 26
   tttggaagat ctttactgat tattcatgcc cttgggagg 39
<210> 27
   <211> 34
   <212> ADN
   <213> Artificial Sequence
<220>
   <223> Description of Artificial Sequence: oligonucléotide
<400> 27
   ttttctgcag atgtccactc gtggcgatct tggg 34
<210> 28
   <211> 40
   <212> ADN
   <213> Artificial Sequence
<220>
   <223> Description of Artificial Sequence: oligonucléotide
<400> 28
   atagtttagc ggccgcttag acaagatttg tttcagtatc 40
<210> 29
   <211> 34
   <212> ADN
   <213> Artificial Sequence
<220>
<223> Description of Artificial Sequence: oligonucléotide
<400> 29
   ttttctgcag atgagacgat ataggatggg acgc 34
<210> 30
   <211> 34
   <212> ADN
   <213> Artificial Sequence
<220>
   <223> Description of Artificial Séquence: oligonucléotide
<400> 30
   agtttagcgg ccgcttataa tcgccgggga tgag 34
<210> 31
   <211> 24
   <212> ADN
   <213> Artificial Sequence
<220>
   <223> Description of Artificial Sequence: oligonucléotide
<400> 31
   gttaaatgtg taccacggga cggg 24
<210> 32
   <211> 41
   <212> ADN
   <213> Artificial Sequence
<220>
   <223> Description of Artificial Sequence: oligonucléotide
<400> 32
   agtttagcgg ccgcttattc aggggacgcg tcgtagactt g 41
<210> 33
   <211> 35
   <212> ADN
   <213> Artificial Séquence
<220>
   <223> Description of Artificial Sequence: oligonucléotide
<400> 33
   tttctgcaga tgtccacttg ttgccgtgct atttg 35
<210> 34
   <211> 31
   <212> ADN
   <213> Artificial Sequence
<220>
   <223> Description of Artificial Sequence: oligonucléotide
<400> 34
   ttttctagat taaaccattt tttcgctttc c 31
<210> 35
   <211> 32
   <212> ADN
   <213> Artificial Sequence
<220>
   <223> Description of Artificial Sequence: oligonucléotide
<400> 35
   tttgtcgaca tgggtttggt aaatataatg cg 32
<210> 36
   <211> 33
   <212> ADN
   <213> Artificial Sequence
<220>
   <223> Description of Artificial Sequence: oligonucléotide
<400> 36
   tttggatcct tagaagtctg ctttcttgta ggg 33
<210> 37
   <211> 33
   <212> ADN
   <213> Artificial Sequence
<220>
   <223> Description of Artificial Sequence: oligonucléotide
<400> 37
   tttgtcgaca tgtctacctt caagcctatg atg 33
<210> 38
   <211> 33
   <212> ADN
   <213> Artificial Sequence
<220>
   <223> Description of Artificial Sequence: oligonucléotide
<400> 38
   tttggatcct tacggaagct gagtatattt gac 33
<210> 39
   <211> 30
   <212> ADN
   <213> Artificial Sequence
<220>
   <223> Description of Artificial Sequence: oligonucléotide
<400> 39
   ttctgcagat gtcctctggt tgccgttcgt 30
<210> 40
   <211> 30
   <212> ADN
   <213> Artificial Sequence
<220>
   <223> Description of Artificial Sequence: oligonucléotide
<400> 40
   tttctagatt aaaccatttt ttcattttcc 30
<210> 41
   <211> 31
   <212> ADN
   <213> Artificial Sequence
<220>
   <223> Description of Artificial Sequence: oligonucléotide
<400> 41
   ttgtcgacat gtggttgcct aatctcgtga g 31
<210> 42
   <211> 33
   <212> ADN
   <213> Artificial Sequence
<220> <223> Description of Artificial Sequence: oligonucléotide
<400> 42
   ttggatccct aaaagtcaga cttcttgtac ggc 33
<210> 43
   <211> 33
   <212> ADN
   <213> Artificial Sequence
<220>
   <223> Description of Artificial Sequence: oligonucléotide
<400> 43
   ttgtcgacat gtctaccttc aagcttatga tgg 33
<210> 44
   <211> 32
   <212> ADN
   <213> Artificial Sequence
<220>
   <223> Description of Artificial Sequence: oligonucléotide
<400> 44
   ttggatcctt acggaagctg ggtatattta ac 32
<210> 45
   <211> 36
   <212> ADN
   <213> Artificial Sequence
<220>
   <223> Description of Artificial Sequence: oligonucléotide
<400> 45
   ttttggtcta gattagtcca cgttgacaac gctgtc 36
<210> 46
   <211> 23
   <212> ADN
   <213> Artificial Sequence
<220>
   <223> Description of Artificial Sequence: oligonucléotide
<400> 46
   cgcaagctta tcgagccgtg cgc 23
<210> 47
   <211> 24
   <212> ADN
   <213> Artificial Sequence
<220>
   <223> Description of Artificial Sequence: oligonucléotide
<400> 47
   gtatcaatcc cagctgaccc cgac 24
<210> 48
   <211> 41
   <212> ADN
   <213> Artificial Sequence
<220>
   <223> Description of Artificial Sequence: oligonucléotide
<400> 48
   aattttggat ccttagccgt ccgggtaacc ctctatgatg c 41
<210> 49
   <211> 24
   <212> ADN
   <213> Artificial Sequence
<220>
   <223> Description of Artificial Sequence: oligonucléotide
<400> 49
   ttttccgtaa caattccgag cagc 24
<210> 50
   <211> 39
   <212> ADN
   <213> Artificial Sequence
<220>
   <223> Description of Artificial Sequence: oligonucléotide
<400> 50
   aattttggat ccttacgtag agttgctatt agacgctgg 39
<210> 51
   <211> 24
   <212> ADN
   <213> Artificial Sequence
<220>
   <223> Description of Artificial Séquence: oligonucléotide
<400> 51
   aacaacagag ggtcgataga aggc 24
<210> 52
   <211> 39
   <212> ADN
   <213> Artificial Sequence
<220>
   <223> Description of Artificial Sequence: oligonucléotide
<400> 52
   aatttttcta gattacacgt tgaccacgct gtcgatgtc 39
<210> 53
   <211> 24
   <212> ADN
   <213> Artificial Sequence
<220>
   <223> Description of Artificial Sequence: oligonucléotide
<400> 53
   gatccggagg aggaatacac accc 24
<210> 54
   <211> 39
   <212> ADN
   <213> Artificial Sequence
<220>
   <223> Description of Artificial Sequence: oligonucléotide
<400> 54
   aattttggat ccctaaaccg gcctgtcctc aacaatcgg 39
<210> 55
   <211> 24
   <212> ADN
   <213> Artificial Sequence
<220>
   <223> Description of Artificial Séquence: oligonucléotide
<400> 55
   cggtttcttg gtgaattcaa cttc 24
<210> 56
   <211> 42
   <212> ADN
   <213> Artificial Sequence
<220>
   <223> Description of Artificial Sequence: oligonucléotide
<400> 56
   aattttggat ccttacgtag agttgctctt agacgttttt gg 42
<210> 57
   <211> 38
   <212> ADN
   <213> Artificial Sequence
<220>
   <223> Description of Artificial Sequence: oligonucléotide
<400> 57
   aaaaacgcgt cgacatgggt actataattc aatttctg 38
<210> 58
   <211> 38
   <212> ADN
   <213> Artificial Sequence
<220>
   <223> Description of Artificial Sequence: oligonucléotide
<400> 58
   ttttctagtc tagattattt atgataaaca aaattctc 38
<210> 59
   <211> 41
   <212> ADN
   <213> Artificial Sequence
<220>
   <223> Description of Artificial Sequence: oligonucléotide
<400> 59
   ttttctagtc tagattagta tgtgtcactt tgtgctaagt g 41
<210> 60
   <211> 41
   <212> ADN
   <213> Artificial Sequence
<220>
   <223> Description of Artificial Sequence: oligonucléotide
<400> 60
   aaaaacgcgt cgacatggtt gcagaagatg cccctgttag g 41
<210> 61
   <211> 35
   <212> ADN
   <213> Artificial Sequence
<220>
   <223> Description of Artificial Sequence: oligonucléotide
<400> 61
   ttttggaaga tctttaggat agtgtcacct gacgg 35
<210> 62
   <211> 37
   <212> ADN
   <213> Artificial Sequence
<220>
   <223> Description of Artificial Sequence: oligonucléotide
<400> 62
   ttaaaagaat tcgacccaaa agcaaatcat gagccac 37
<210> 63
   <211> 33
   <212> ADN
   <213> Artificial Sequence
<220>
   <223> Description of Artificial Sequence: oligonucléotide
<400> 63
   ttaaaaggcc tttaggatag tgtcacctga cgg 33
<210> 64
   <211> 33
   <212> ADN
   <213> Artificial Sequence
<220>
   <223> Description of Artificial Sequence: oligonucléotide
<400> 64
   tatgcggccg ccaccatgtg gctgcagaac ctg 33
<210> 65
   <211> 36
   <212> ADN
   <213> Artificial Sequence
<220>
   <223> Description of Artificial Sequence: oligonucléotide
<400> 65
   tatgcggccg ctacgtatca cttcttgact ggtttc 36
<210> 66
   <211> 435
   <212> ADN
   <213> Canis sp.
<400> 66
   atgtggctgc agaacctgct tttcttgggc actgtggtct gcagcatctc tgcacccacc 60
   cgctcaccca cccttgtcac tcggccctct cagcacgtgg atgccatcca ggaagccctg 120
   agccttttga acaacagtaa tgacgtgact gctgtgatga ataaagcagt aaaagtggtc 180
   tctgaagtgt ttgaccctga ggggccaaca tgcctggaga cccgcctaca gctgtacaag 240
   gagggcctgc agggcagcct caccagcctc aagaatccct taaccatgat ggccaatcac 300
   tataagcagc actgtccccc taccccggaa tctccctgtg caacccagaa tattaacttc 360
   aaaagtttca aagagaacct gaaggatttt ctgtttaaca tcccctttga ctgctggaaa 420
   ccagtcaaga agtga                                                  435
<210> 67
   <211> 435
   <212> ADN
   <213> Felis catus
<400> 67
   atgtggctgc agaacctgct tttcctgggc actgtggtct gcagcatctc tgcacccacc 60
   agttcaccca gctctgtcac tcggccctgg caacacgtgg atgccatcaa ggaggctctg 120
   agccttctga acaacagtag tgaaataact gctgtgatga atgaagcagt agaagtcgtc 180
   tctgaaatgt ttgaccctga ggagccgaaa tgcctgcaga ctcacctaaa gctgtacgag 240
   cagggcctac ggggcagcct catcagcctc aaggagcctc tgagaatgat ggccaaccat 300
   tacaagcagc actgcccctt tactccggaa acgccctgtg aaacccagac tatcaccttc 360
   aaaaatttca aagagaatct gaaggatttt ctgtttaaca tcccctttga ctgctggaaa 420
   ccagtcaaga agtga                                                  435
<210> 68
   <211> 435
   <212> ADN
   <213> Felis catus
<400> 68
   atgtggctgc agaacctgct tttcctgggc actgtggtct gcagcatctc tgcacccacc 60
   agttcaccca gctctgtcac tcggccctgg caacacgtgg atgccatcaa ggaggctctg 120
   agccttctga acaacagtag tgaaataact gctgtgatga atgaagcagt agaagtcgtc 180
   tctgaaatgt ttgaccctga ggagccgaaa tgcctgcaga ctcacctaaa gctgtacgag 240
   cagggcctac ggggcagcct catcagcctc aaggagcctc tgaggatgat ggccaaccat 300
   tacaagcagc actgccccct tactccggaa acgccctgtg aaacccagac tatcaccttc 360
   aaaaatttca aagagaatct gaaggatttt ctgtttaaca tcccctttga ctgctggaaa 420
   ccagtcaaga agtga                                                  435
<210> 69
   <211> 435
   <212> ADN
   <213> Equus sp.
<400> 69
   atgtggctgc agaacctgct tcttctgggc actgtggttt acagcatgcc cgcacccacc 60
   cgccaaccca gccctgtcac tcggccctgg cagcatgtgg atgccatcaa ggaggccctg 120
   agccttctga acaacagtag tgacactgct gctatcatga atgaaacagt agaagtcgtc 180
   tctgaaacgt ttgacgccga ggagctgaca tgcctgcaga ctcgcctgaa gctgtacaaa 240
   cagggcttgc ggggcagcct catcaagctc gaaggcccct tgaccatgat ggccagccac 300
   tacaagcagc actgcccccc caccctggaa acttcctgtg caacccagat gatcaccttc 360
   aaaagtttca aaaagaacct gaaggatttt ctgtttgaga tcccgtttga ctgctggaag 420
   ccagcccaga agtaa                                                  435
<210> 70
   <211> 44
   <212> ADN
   <213> Artificial Sequence
<220>
   <223> Description of Artificial Sequence: oligonucléotide
<400> 70
   catcatcatg tcgacgccac catgtggctg cagaacctgc ttct 44
<210> 71
   <211> 41
   <212> ADN
   <213> Artificial Sequence
<220>
   <223> Description of Artificial Sequence: oligonucléotide
<400> 71
   catcatcatg cggccgctac ttctgggctg ctggcttcca g 41
<210> 72
   <211> 1590
   <212> ADN
   <213> canine parainfluenza virus
<400> 72
   atgggtacta taattcaatt tctggtggtc tcctgtctat tggcaggagc aggcagcctt 60
   gatctagcag ccctcatgca aatcggtgtc attccaacaa atgtccggca acttatgtat 120
   tatactgagg cctcatcagc attcattgtt gtgaagttaa tgcctacaat tgactcgccg 180
   attagtggat gtaatataac atcaatttca agctataatg caacagtgac aaaactccta 240
   cagccgatcg gtgagaattt ggaaacgatt aggaaccagt tgattccaac tcggaggaga 300
   cgccggtttg caggggtggt gattggatta gctgcattag gagtagctac tgccgcacag 360
   gtcactgccg cagtagcact agtaaaggca aataaaaatg ctgcggctat actcaatctc 420
   aaaaatgcaa tccaaaaaac aaatacagca gttgcagatg tggtccaggc cacacaatca 480
   ctaggaacgg cagttcaagc agttcaagat cacataaaca gtgtggtaag tccagcaatt 540
   acagcagcca attgtaaggc ccaagatgct atcattggct caatcctcaa tctctatttg 600
   accgagttga caactatctt ccacaatcaa attacaaacc ctgcattgag tcctattaca 660
   attcaagctt taaggatcct actggggagt accttgccga ctgtggtcga aaaatctttc 720
   aatacccaga taagtgcagc tgagcttctc tcatcagggt tattgacagg ccagattgtg 780
   ggattagatt tgacctatat gcagatggtc ataaaaattg agctgccaac tttaactgta 840
   caacctgcaa cccagatcat agatctggcc accatttctg cattcattaa caatcaagaa 900
   gtcatggccc aattaccaac acgtgttatt gtgactggca gcttgatcca agcctatccc 960
   gcatcgcaat gcactattac acccaacact gtgtactgta ggtataatga tgcccaagta 1020
   ctctcagatg atacgatggc ttgcctccaa ggtaacttga caagatgcac cttctctcca 1080
   gtggttggga gctttctcac tcgattcgtg ctgttcgatg gaatagttta tgcaaattgc 1140
   aggtcgatgt tatgcaagtg catgcagcct gctgctgtga tcctacagcc gagttcatcc 1200
   cctgtaactg tcattgacat gtacaaatgt gtgagtctgc agcttgacaa tctcagattc 1260
   accatcactc aattggccaa tgtaacctac aatagcacca tcaagcttga aacatcccag 1320
   atcttgccta ttgatccgtt ggatatatcc cagaatctag ctgcggtgaa taagagtcta 1380
   agtgatgcac tacaacactt agcacaaagt gacacatacc tttctgcaat cacatcagct 1440
   acgactacaa gtgtattatc cataatggca atctgtcttg gatcgttagg tttaatatta 1500
   ataatcttgc tcagtgtagt tgtgtggaag ttattgacca ttgtcactgc taatcgaaat 1560
   agaatggaga attttgttta tcataaataa                                 1590
<210> 73
   <211> 1698
   <212> ADN
   <213> canine parainfluenza virus
<400> 73
   atggttgcag aagatgcccc tgttaggggc acttgccgag tattatttcg aacaacaact 60
   ttaatttttc tatgcacact actagcatta agcatctcta tcctttatga gagtttaata 120
   acccaaaagc aaatcatgag ccacgcaggc tcaactggat ctaattctag attaggaagt 180
   atcactgatc ttcttaataa tattctctct gtcgcaaatc agattatata taactctgca 240
   gtcgctctac ctctacaatt ggacactctt gaatcaacac tccttacagc cattaagtct 300
   cttcaaacca gtgacaagct agaacagaac tgctcgtggg gtgctgcact gattaataat 360
   aatagataca ttaatggcat caatcagttc tatttctcaa ttgctgaggg tcgcaatctg 420
   acacttggcc cacttcttaa tatacctagt ttcattccaa ctgccacgac accagagggc 480
   tgcaccagga tcccatcatt ctcgctcacc aagacacact ggtgttatac acacaatgtt 540
   atcctgaatg gatgccagga tcatgtatcc tcaaatcaat ttgtttccat gggaatcatt 600
   gaacccactt ctgccgggtt tccatccttt cgaaccctaa agactctata tctcagcgat 660
   ggggtcaatc gtaagagctg ctctatcagt acagttccgg ggggttgtat gatgtactgt 720
   tttgtctcta ctcaaccaga gagggatgac tacttttcta ccgctcctcc agaacaacga 780
   attattataa tgtactataa tgatacaatc gtggagcgca taattaatcc acccggggta 840
   ctagatgtat gggcaacatt gaccccagga acaggaagcg gggtatatta tttaggttgg 900
   gtgctctttc caatatatgg cggcgtgatt aaagatacga gtttatggaa taatcaagca 960
   aataaatact ttatccccca gatggttgct gctctctgct cacaaaacca ggcaactcaa 1020
   gtccaaaatg ctaagtcatc atactatagc agctggtttg gcaatcgaat gattcagtct 1080
   gggatcctgg catgtcctct tcaacaggat ctaaccaatg agtgtttagt tctgcccttt 1140
   tctaatgatc aggtgcttat gggtgctgaa gggagattat acatgtatgg tgactcggtg 1200
   tattactacc aaagaagcac tagttggtgg cctatgacca tgctgtataa ggtaaccata 1260
   acattcacta atggtcagcc atctgctata tcagctcaga atgtgcccac acagcaggtc 1320
   cctagacctg ggacaggagg ctgctctgca acaaatagat gtcccggttt ttgcttgaaa 1380
   ggagtgtatg ctgatgcctg gttactgacc aacccttcgt ctaccagtac atttggatca 1440
   gaagcaacct tcactggttc ttatctcaac gcagcaactc agcgtatcaa tccgacgatg 1500
   tatatcgcga acaacacaca gatcataagc tcacagcaat ttggatcaag cggtcaagaa 1560
   gcagcatata gccacacaac ttgttttagg gacacaggct ctgttatggt atactgtatc 1620
   tatattattg aattgtcctc atctctctta ggacaatttc agattgtccc atttatccgt 1680
   caggtgacac tatcctaa                                                 1698